(19) **Europäisches Patentamt / European Patent Office / Office européen des brevets**

(11) **EP 2 285 823 B2**

(12) **NEW EUROPEAN PATENT SPECIFICATION**
After opposition procedure

(45) Date of publication and mention
of the opposition decision:
**06.03.2024 Bulletin 2024/10**

(45) Mention of the grant of the patent:
**27.04.2016 Bulletin 2016/17**

(21) Application number: **09763466.1**

(22) Date of filing: **09.06.2009**

(51) International Patent Classification (IPC):
*C12N 9/28* (2006.01)    *C12N 9/34* (2006.01)
*A23K 40/10* (2016.01)    *A23K 40/30* (2016.01)
*C12N 9/00* (2006.01)    *C12N 9/54* (2006.01)
*C12N 9/98* (2006.01)    *C11D 3/386* (2006.01)
*A23L 29/00* (2016.01)    *A23K 20/189* (2016.01)

(52) Cooperative Patent Classification (CPC):
**C12N 9/98; A23K 20/189; A23K 40/10;**
**A23K 40/30; A23L 29/06; C11D 3/38672;**
**C12N 9/00; C12N 9/2417; C12N 9/2428;**
**C12N 9/54;** C12Y 302/01001; C12Y 302/01003;
C12Y 304/21062

(86) International application number:
**PCT/US2009/046783**

(87) International publication number:
**WO 2009/152176 (17.12.2009 Gazette 2009/51)**

(54) **RECOVERY OF INSOLUBLE ENZYME FROM FERMENTATION BROTH AND FORMULATION OF INSOLUBLE ENZYME**

RÜCKGEWINNUNG VON UNLÖSLICHEM ENZYM AUS EINER FERMENTATIONSBRÜHE UND FORMULIERUNG DES UNLÖSLICHEN ENZYMS

RÉCUPÉRATION D'ENZYMES INSOLUBLES À PARTIR D'UN BOUILLON DE FERMENTATION ET FORMULATION D'ENZYMES INSOLUBLES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **09.06.2008 US 60087 P**

(43) Date of publication of application:
**23.02.2011 Bulletin 2011/08**

(73) Proprietor: **Danisco US Inc.**
**Palo Alto, CA 94304 (US)**

(72) Inventors:
• **BODO, Michael**
**Palo Alto, California 94304 (US)**
• **CHRISTENSEN, Robert, I.**
**Palo Alto, California 94304 (US)**
• **DHALIWAL, Rajdeep, S.**
**Palo Alto, California 94304 (US)**
• **HENG, Meng, H.**
**Palo Alto, California 94304 (US)**

(74) Representative: **Mewburn Ellis LLP**
**Aurora Building**
**Counterslip**
**Bristol BS1 6BX (GB)**

(56) References cited:
WO-A1-01/25411    WO-A2-97/12958
WO-A2-2004/003187    US-A- 5 879 920
US-B1- 6 316 240

• FAHNERT BEATRIX ET AL: "INCLUSION BODIES: FORMATION AND UTILISATION" 1 January 2004 (2004-01-01), ADVANCES IN BIOCHEMICAL ENGINEERING, BIOTECHNOLOGY, SPRINGER, BERLIN, DE, PAGE(S) 93 - 142 , XP008069598 ISSN: 0724-6145 page 103, paragraph 1 page 121, paragraph 3 - paragraph 4
• WERNER R G ET AL: "PURIFICATION OF PROTEINS PRODUCED BY BIOTECHNOLOGICAL PROCESS" ARZNEIMITTEL FORSCHUNG. DRUG RESEARCH, ECV EDITIO CANTOR VERLAG, AULENDORF, DE, vol. 38, no. 3, 1 January 1988 (1988-01-01), pages 422-428, XP002150296 ISSN: 0004-4172

EP 2 285 823 B2

- SAEKI ET AL: "Detergent alkaline proteases: enzymatic properties, genes, and crystal structures" JOURNAL OF BIOSCIENCE AND BIOENGINEERING, ELSEVIER, AMSTERDAM, NL, vol. 103, no. 6, 1 June 2007 (2007-06-01), pages 501-508, XP022153072 ISSN: 1389-1723
- ITO SUSUMU ET AL: "Akaline detergent enzymes from alkaliphiles: Enzymatic properties, genetics and structures" EXTREMOPHILES, SPRINGER VERLAG, TOKYO, JP, vol. 2, no. 3, 1 August 1998 (1998-08-01), pages 185-190, XP002503766 ISSN: 1431-0651

**Description**

**FIELD OF THE INVENTION**

The invention relates to methods for making an enzyme-containing granular formulation comprising recovered insoluble enzyme and microbial cells and/or cell debris.

**BACKGROUND**

Conventional recovery of enzymes from microbial fermentation broth for industrial uses in liquid or solid product forms typically involves several processing steps. Most conventional recovery processes include four similar steps which occur sequentially: removal of non-product insoluble components of microbial broth; isolation of products; purification; and polishing. (See, *e.g.,* Enzyme in Industry: Production and Applications (2007) Wolfgang Aehle, ed., 3rd edition, Wiley-VCH, p. 49; Bioseparations: Science and Engineering (2003) R.G. Harrison, P. Todd, S.R. Rudgei. D.P. Petrides, Oxford University Press, p. 32.) For example, recovery and formulation of an intracellularly produced enzyme generally includes the following processing steps: (1) cell lysis; (2) pretreatment; (3) cell separation; (4) concentration; and (5) formulations - (a) chemical addition for liquid stabilization, followed by polish filtration; or (b) chemical addition for solid stabilization, followed by drying.

Cell lysis releases the enzyme(s) of interest from the cells by breaking the cell walls. Cell lysis may be achieved by contacting the cells with an enzyme that degrades cell walls such as lysozyme, by inducing fermentation environment changes such as alteration in pH, temperature, dissolved oxygen concentration, glucose concentration, salt concentration, or concentration of other additives to cause spontaneous lysis, by mechanical disruption of the cell wall such as by homogenization, or by a combination of these methods. For enzyme expressed extracellularly (secreted into the culture medium), cell lysis is optional. (See, *e.g.*, Isolation and Purification of Proteins (2003) R. Hatti-Kaul and B. Mattiasson, ed., Marcell Dekker, Inc., pp. 1-27.)

A cell separation step is employed after cell lysis to produce a clarified solution containing the enzyme(s) of interest. Typically, cell separation involves centrifugation and/or filtration, to remove cell debris and any insoluble material produced during and/or after fermentation, and/or as a result of cell lysis. Suitable centrifugation methods include disk stack or decanter centrifugation. Suitable filtration methods include rotary vacuum filtration, filtration through a plate-and-frame filter press, or filtration through a membrane micro-filter.

Cell separation using centrifugation is often a compromise between clarity and throughput. It is common practice to use high throughput centrifugation and then remove trace amounts of suspended solids in amounts sufficient to interfere with subsequent processing steps via high throughput filtration. Most filtration methods in practice today require the use of processing aids such as diatomaceous earth or the like and carbon (PCT Application No. WO01/87468) to achieve separation efficiency. Diatomaceous earth improves filtration process throughput and filtrate clarity.

A pretreatment step is typically employed before physical separation of cells or cell debris from the liquid containing the enzyme(s) of interest. Separating cell debris without such a step is generally non-economical and time consuming due to the small particle size of debris and viscous nature of lysed fermentation broth. Often, pretreatment includes introduction of flocculant(s) to the lysed or unlysed broth. Polyelectrolytes have received much attention as a method to enhance cell flocculation in bacterial cell removal from fermentation broth. Baran (1988) Colloids Surf. 31:259-264; Bautista et al. (1986) Biotechnol. Lett. 8:315-318; Chen and Berg (1993) Chem. Eng. Sci. 48:1775-1784; Cumming et al. (1996) Biotechnol. Tech. 4:55-60; Hustedt and Theelen (1989) DeChema Biotechnology Conferences - VCH Veragsgesselschaft 3:1071-1075; Ramsden et al. (1998) Biotechnol. Tech. 12:599-603; Shan et al. (1996) J. Biotechnol. 49:173-178.

Flocculation facilitates the removal of cell debris from the liquid portion containing the enzyme(s) of interest. Some commonly used flocculants include polyethyleneimine, polydiallyldimethyl ammonium chloride, and methacryloyloxyethyl trimethylanunonium chloride-acrylamide copolymer. Flocculation of cell debris is a charge-based phenomenon, requiring low ionic strength for effectiveness. As a result, dilution of the fermentation broth by water is generally required to flocculate the cell debris.

Depending on the desired final product concentration and purity, the clarified liquid containing the enzyme(s) of interest from the cell separation step may meet the requirements for formulations to produce the product. However, further processing is often required to increase the concentration of enzyme before formulation. Concentration involves dewatering, which is typically achieved by ultrafiltration or evaporation for heat stable enzymes. Other methods for concentration include precipitation, crystallization, and chromatography. (*See, e.g.,* PCT Application No. WO 91/09941.) These are not common practices for industrial enzyme recovery due to the cost involved.

As outlined above, conventional recovery and formulation of enzymes includes multiple steps. Each step added to a manufacturing process decreases the overall process yield and adds cost in the form of energy, time, and labor.

Advances in expression technology have resulted in the ability to obtain relatively high enzyme concentrations (*e.g.,* 10-100 g/l) in fermentation broth. In some cases, the expression level exceeds the solubility limit of an enzyme of interest, and the enzyme is present in a precipitated or crystalline form at the end of fermentation. When a traditional recovery process as described above is used to recover the enzyme from the fermentation broth, enzyme precipitates will be removed simultaneously with insoluble cell debris and other insoluble material during the cell separation step. As a result, the recovery yield will be low. One proposed solution to this problem includes addition of a polyol immediately after a recovery step to a supersaturated enzyme solution to prevent its precipitation. (European Patent No. EP1417301) However, such a process requires additional material costs and adds an extra step to the recovery process. A method for recovering insoluble enzyme without addition of a polyol would be advantageous.

Enzyme-containing granules are incorporated into products in several industries, including detergent, textile-processing, food (*e.g.,* baking), animal feed, and fuel ethanol industries. Such granules may be prepared by a number of technologies, including fluidized bed spray coating, high sheer granulation, extrusion, spheronization, prilling, and spray drying.

Traditionally, such enzyme-containing granules contain enzymes that are in soluble form before incorporation into the granules, and if expressed in a microbial host, are at least partially purified to remove microbial cells and/or cell debris produced from lysis of the cells.

In cases where insoluble enzyme is present in the microbial broth, solubilization is required before recovery. In some cases this can be achieved easily by changing pH and/or temperature or by addition of simple salts. In other cases, addition of polyols or sugars is necessary. Subsequent to solubilization, cell separation can be performed. The resulting clarified stream is generally too dilute to be used for formulation. A concentration step, typically by ultrafiltration, is used. The sugar that was added will freely pass the ultrafiltration membrane and become a waste stream. The resulting concentrate generally can be formulated into liquid product. However, it is not always amenable to granulation. As a result, removal of the sugar prior to granulation is necessary. This can be achieved by diafiltration, whereby more waste is generated. The impact of this approach is significant cost increase for both liquid and solid products, by virtue of processing steps and waste disposal. This invention provides an alternate way of way of producing product economically from such broth.

There is a need for granular enzyme formulations in which the enzyme is in insoluble form and in which cells and/or insoluble cell components are not removed.

## BRIEF SUMMARY OF THE INVENTION

The invention provides methods for recovery and formulation of insoluble enzymes.

In one aspect, a method is provided for making an enzyme-containing granular formulation, comprising recovering insoluble enzyme from a microbial broth that comprises microbial cells that express the enzyme and/or cell debris from microbial cells that express the enzyme, without removing the microbial cells and/or cell debris, thereby producing a composition comprising recovered insoluble enzyme and microbial cells and/or cell debris., wherein at last some of the enzyme is insoluble in the microbial broth, and producing an enzyme-containing granule that comprises the composition. The insoluble enzyme is enzymatically active in the granule (*i.e.,* the enzyme is catalytically active in the solid state or has catalytic potential and becomes catalytically active when solubilized in an appropriate solvent and under appropriate conditions for catalysis to occur)

The method may comprise adding a precipitant to a microbial broth comprising a soluble enzyme expressed by the microbial cells, thereby causing at least some of the enzyme to become insoluble; or expressing an enzyme in microbial cells in a fermentation medium, wherein at least some of the enzyme in insoluble in the fermentation medium.

In some embodiments, recovering includes at least one recovery operation selected from broth conditioning; cell lysis; homogenization; diafiltration; and solid-liquid separation. In some embodiments, recovering includes two or more recovery operations selected from broth conditioning; cell lysis; homogenization; diafiltration; and solid liquid separation, performed in any order.

In some embodiments, recovering insoluble enzyme comprises removing at least part of the liquid phase of the microbial broth. In some embodiments, removing at least part of the liquid phase of the microbial broth comprises concentrating the microbial broth solids. In some embodiments, concentrating the microbial broth comprises a membrane filtration process selected from ultrafiltration, microfiltration, reverse osmosis, and nanofiltration. In some embodiments, removing at least part of the liquid phase of the microbial broth, e.g., concentrating the microbial broth, comprises a process selected from rotary drum vacuum filtration, plate and frame filtration, belt filtration, centrifugation, cyclone separation, decantation, and evaporation.

In some embodiments, recovering insoluble enzyme comprises diafiltration.

In some embodiments, the microbial broth is produced by growing a microbial cell that expresses the enzyme in a growth medium under conditions suitable for expression of the enzyme, wherein the enzyme is insoluble under the growth conditions. In other embodiments, the microbial broth is produced by growing a microbial cell that expresses the enzyme in a growth medium under conditions suitable for expression of the enzyme, wherein the enzyme is soluble under the growth conditions, and wherein a precipitant is added prior to recovering the enzyme to render at least some of the enzyme insoluble in the microbial broth. In some embodiments, the precipitant is selected from adjustment of pH, adjustment of temperature, addition of salt, addition of acid, addition of base, addition of at least one other protein, addition of buffer, sand addition of polyelectrolyle, or a combination thereof. In one embodiment, insoluble enzyme is produced by adding a precipitant to a microbial broth comprising a soluble enzyme, thereby rendering at least some of the enzyme insoluble in the microbial broth, wherein the precipitant s selected from adjustment of pH, adjustment of temperature, addition of salt, addition of acid, addition of base, addition of at least one other protein, addition of buffer, and addition of polyelectrolyte, or a combination thereof.

In some embodiments, the composition comprising recovered insoluble enzyme comprises intact microbial cells. In one embodiment, the intact microbial cells are live microbial cells. In another embodiment, the intact microbial cells are dead microbial cells. In other embodiments, the composition comprising recovered insoluble enzyme comprises lysed microbial cells.

In some embodiments in which the expressed enzyme is insoluble in the microbial broth, the method comprises disrupting microbial cell membranes to produce a microbial cell lysate, before or after recovery of insoluble enzyme. In other embodiments in which the expressed enzyme is soluble in the microbial broth, the method comprises disrupting microbial cell membranes before or after addition of a precipitant, prior to recovery of precipitated enzyme.

In some embodiment, the microbial broth comprising microbial cells and/or cell debris comprises a microbial cell lysate, produced by disrupting microbial cell membranes of intact microbial cells.

In some embodiments, disrupting microbial cell membranes comprises contacting the cell with an enzyme that is capable of effecting microbial cell lysis. In one embodiment, the enzyme that is capable of effecting microbial cell lysis is a lysozyme enzyme. In other embodiments, disrupting microbial cell membranes comprises a mechanical cell membrane disruption process, such as homogenization. In some embodiments, disrupting microbial cell membranes comprises a mechanical shear process, for example, selected from homogenization; high shear mixing; pressure extrusion; and high shear pumping. In some embodiments, the method further comprises homogenizing the microbial cell lysate to produce a homogenized microbial cell lysate prior to or after recovering insoluble enzyme.

In some embodiments, the method further comprises diafiltering the microbial cell lysate prior to or after recovering insoluble enzyme. In some embodiments, the method further comprises homogenizing and diafiltering the microbial cell lysate prior or after to recovering insoluble enzyme.

In some embodiments, the microbial cells are bacterial cells. In some embodiments, the bacterial cells are *Bacillus* cells. In some embodiments, the *Bacillus* cells are *Bacillus subtilis* or *Bacillus licheniformis* cells.

In some embodiments, the microbial cells are fungal cells. In some embodiments, the fungal cells are *Trichoderma* cells. In some embodiments, the *Trichoderma* cells are *Trichoderma reesei* cells.

In some embodiments, at least about any of 10, 20, 30, 40, 50, 60, 70, 80, or 90% of the enzyme is insoluble in the microbial broth.

In some embodiments of the recovery methods, the purity of the insoluble enzyme is increased by at least about 10%.

Also described herein is an enzyme containing granule, comprising a composition produced according to a method for recovering an insoluble enzyme from a microbial broth as described herein, wherein the composition comprises recovered insoluble enzyme and microbial cells and/or cell debris. The composition may further comprise at least one formulation ingredient.

In one embodiment, the enzyme-containing granule is produced in a top-spray fluid bed processor. In some embodiments, the enzyme-containing granule is produced by a granulation process selected from top-spray fluid bed processing; bottom spray Wurster coater processing; high shear granulation; extrusion; and pan coating.

In one embodiment, the method of making granule comprises coating an enzyme-containing layer comprising the composition comprising insoluble enzyme, e.g., recovered insoluble enzyme, and microbial cells and/or cell debris onto a core in said top-spray fluid bed processor. The method optionally includes coating a salt layer between the core and the enzynie-containing layer. In one embodiment, the method further comprises coating a layer comprising a barrier salt over said enzyme-containing layer. In one embodiment, the method further comprises coating an outer coating layer comprising a polymer and/or pigment over said barrier salt layer.

In one embodiment, the method comprises coating a barrier salt layer and/or one or more coating layers over an enzyme-containing core comprising insoluble enzyme, e.g., recovered insoluble enzyme, and microbial cells and/or cell debris. In one embodiment, the enzyme-containing core is produced by top-spray fluid bed processing. In one embodiment, the enzyme-containing core is produced by high shear granulation.

Also described herein is an enzyme-containing granule comprising: a core; optionally, a salt layer coated over the core; and an enzyme-containing layer coated over the core or salt layer, wherein the enzyme-containing layer comprises a composition comprising recovered insoluble enzyme, and microbial cells and/or cell debris, produced according to the methods set out in the claims. The enzyme is enzymatically active in the granule (*i.e.*, the enzyme is catalytically active in the solid state or has catalytic potential and becomes catalytically active when solubilized in an appropriate solvent and under appropriate conditions for catalysis to occur).

## BRIEF DESCRIPTION OF THE DRAWINGS

The granule may further comprise a barrier salt layer coated over said enzyme-containing layer. The granule may further comprise an outer coating layer coated over said barrier salt layer, wherein said outer coating layer comprises a polymer and/or pigment.

Also described herein is an enzyme-containing granule comprising an enzyme-containing core and a barrier salt layer and/or one or more coating layers surrounding the core, wherein the core comprises a composition comprising insoluble enzyme and microbial cells and/or cell debris produced according to the method set out in the claims. The enzyme is enzymatically active in the granule (i.e., the enzyme is catalytically active in the solid state or has catalytic potential and becomes catalytically active when solubilized in an appropriate solvent and under appropriate conditions for catalysis to occur).

The enzyme-containing core may be produced by top-spray fluid bed processing. The enzyme-containing core may be produced by high shear granulation.

Also described herein are compositions comprising any of the enzyme-containing granules described herein, e.g. a detergent composition, a textile processing composition, an animal feed composition, or a food composition.

Figure 1 shows the wash application activity Performance of enzyme-containing granules prepared as described in Example 4 after storage in detergent, as described in Example 13.

Figure 2 shows the enzymatic stability of granules prepared in Examples 1,2,4, and 6 after storage in detergent.

## DETAILED DESCRIPTION

The invention provides methods for recovering and formulating insoluble enzymes from microbial fermentation broth without removing microbial cells or lysed insoluble cell debris from the broth. Also described herein are granular formulations that contain insoluble enzymes and microbial cells and/or cell debris recovered according to the methods described herein.

The practice of the present invention will employ, unless otherwise indicated, conventional techniques of molecular biology (including recombinant techniques), microbiology, cell biology, and biochemistry, which are within the skill of the art. Such techniques are explained fully in the literature, for example, Molecular Cloning: A Laboratory Manual, second edition (Sambrook et al., 1989); Oligonucleotide Synthesis (M. J. Gait, ed., 1984; Current Protocols in Molecular Biology (P. M. Ausubel et al., eds., 1994); PCR: The Polymerise Chain Redaction (Mullis et al., eds., 1994); and Gene Transfer and Expression: A Laboratory Manual (Kriegler, 1990).

Unless defmed otherwise herein, all technical and scientific terms used herein have the same meaning as commonly understood by one of ordinary skill in the art to which this invention belongs. Singleton, et al., Dictionary of Microbiology and Molecular Biology, second ed., John Wiley and Sons, New York (1994), and Hale & Markham, The Harper Collins Dictionary of Biology, Harper Perennial, NY (1991) provide one of skill with a general dictionary of many of the terms used in this invention. Any methods and materials similar or equivalent to those described herein can be used in the practice or testing of the present invention.

Numeric ranges provided herein are inclusive of the numbers defining the range.

Unless otherwise indicated, nucleic acids are written left to right in 5' to 3' orientation and amino acid sequences are written left to right in amino to carboxy orientation, respectively.

### Definitions

As used herein, the term "polynucleotide" refers to a polymeric form of nucleotides of any length and any three-dimensional structure and single- or multi-stranded (e.g., single-stranded, double-stranded, triple-helical, etc.), which contain deoxyribonucleotides, ribonucleotides, and/or analogs or modified forms of deoxyribonucleotides or ribonucleotides, including modified nucleotides or bases or their analogs. Because the genetic code is degenerate, more than one codon may be used to encode a particular amino acid, and the present invention encompasses polynucleotides which encode a particular amino acid sequence. Any type of modified nucleotide or nucleotide analog may be used, so long as the polynucleotide retains the desired functionality under conditions of use, including modifications that increase nuclease resistance (e.g., deoxy, 2'-O--Me, phosphorothioates, etc.). Labels may also be incorporated for purposes of detection or capture, for example, radioactive or nonradioactive labels or anchors, e.g., biotin. The term polynucleotide also includes peptide nucleic acids (PNA). Polynucleotides may be naturally occurring or non-naturally occurring. The terms "polynucleotide" and "nucleic acid" and "oligonucleotide" are used herein interchangeably. Polynucleotides of the invention may contain RNA, DNA, or both, and/or modified forms and/or analogs thereof. A sequence of nucleotides may be interrupted by non-nucleotide components. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR$_2$ ("amidate"), P(O)R, P(O)OR', CO or CH$_2$ ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. Polynucleotides may be linear or circular or comprise a combination of linear and circular portions.

As used herein, "polypeptide" refers to any composition comprised of amino acids and recognized as a protein by those of skill in the art. The conventional one-letter or three-letter code for amino acid residues is used herein. The terms "polypeptide." and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

As used herein, a "vector' refers to a polynucleotide sequence designed to introduce nucleic acids into one or more cell types. Vectors include cloning vectors, expression vectors, shuttle vectors, plasmids, phage particles, cassettes and the like.

As used herein, the term "expression" refers to the process by which a polypeptide is produced based on the nucleic acid sequence of a gene. The process includes both transcription and translation.

As used herein, "expression vector" refers to a DNA construct containing a DNA coding sequence (*e.g.*, gene sequence) that is operably linked to one or more suitable control sequence(s) capable of effecting expression of the coding sequence in a host. Such control sequences include a promoter to effect transcription, an optional operator sequence to control such transcription, a sequence encoding suitable mRNA ribosome binding sites, and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. The plasmid is the most commonly used form of expression vector. However, the invention is intended to include such other forms of expression vectors that serve equivalent functions and which are, or become, known in the art.

A "promoter' refers to a regulatory sequence that is involved in binding RNA polymerase to initiate transcription of a gene. The promoter may be an inducible promoter or a constitutive promoter. A non-limiting example of an inducible promoter which may be used in the invention is *Trichoderma reesei* cbh1, which is an inducible promoter.

The term "operably linked" refers to juxtaposition wherein the elements are in an arrangement allowing them to be functionally related. For example, a promoter is operably linked to a coding sequence if it controls the transcription of the coding sequence.

"Under transcriptional control" is a term well understood in the art that indicates that transcription of a polynucleotide sequence depends on its being operably linked to an element which contributes to the initiation of, or promotes transcription.

"Under translational control" is a term well understood in the art that indicates a regulatory process which occurs after mRNA has been formed.

A "gene" refers to a DNA segment that is involved in producing a polypeptide and includes regions preceding and following the coding regions as well as intervening sequences (introns) between individual coding segments (exons).

As used herein, the term "host cell" refers to a cell or cell line into which a recombinant expression vector for production of a polypeptide may be transfected for expression of the polypeptide. Host cells include progeny of a single host cell, and the progeny may not necessarily be completely identical (in morphology or in total genomic DNA complement) to the original parent cell due to natural, accidental, or deliberate mutation. A host cell includes cells transfected or transformed *in vivo* with an expression vector. "Host cell" refers to both cells and protoplasts created from the cells of a filamentous fungal strain and particularly a *Trichodermna* sp. strain.

The term "recombinant" when used in reference to a cell, nucleic acid, protein or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

A "signal sequence" refers to a sequence of amino acids bound to the N-terminal portion of a protein which facilitates the secretion of the mature form of the protein from the cell. The mature form of the extracellular protein lacks the signal sequence which is cleaved off during the secretion process.

The term "selective marker' or "selectable marker" refers to a gene capable of expression in a host cell that allows for ease of selection of those hosts containing an introduced nucleic acid or vector. Examples of selectable markers include but are not limited to antimicrobial substances (*e.g.*, hygromycin, bleomycin, or chloramphenicol) and/or genes that confer a metabolic advantage, such as a nutritional advantage, on the host cell.

The term "derived from" encompasses the terms "originated from," "obtained from," "obtainable from," "isolated from," and "created from."

The term "filamentous fungi" refers to all filamentous forms of the subdivision Eumycotina (*See*, Alexopoulos, C. J. (1962), INTRODUCTORY MYCOLOGY, Wiley, New York). These fungi are characterized by a vegetative mycelium with a cell wall composed of chitin, cellulose, and other complex polysaccharides. The filamentous fungi of the present invention are morphologically, physiologically, and genetically distinct from yeasts. Vegetative growth by filamentous fungi is by hyphal elongation and carbon catabolism is obligatory aerobic. A filamentous fungal parent cell herein may be a cell of a species of, but not limited to, *Trichoderma, (e.g., Trichoderma reesei* (previously classified as *T. longibrachiatum* and currently also known as *Hypocrea jecorina*), *Trichoderma viride, Trichoderma koningii, Trichoderma harzianum*); *Penicillium sp., Humicola sp.* (*e.g., Humicola insolens* and *Humicola grisea*); *Chrysosporium sp.* (*e.g., C. lucknowense*), *Gliocladium sp., Aspergillus sp.* (*e.g., A. oryzae, A. niger,* and A. *awamori*), *Fusarium sp., Neurospora sp., Hypocrea sp., and Emericella sp.* (See also, Innis et al., (1985) Sci. 228:21 -26).

As used herein, the term "*Trichoderma*" or "*Trichoderma* sp." refers to any fungal genus previously or currently classified as *Trichroderma.*

The term "culturing" refers to growing a population of microbial cells under suitable conditions for growth, in a liquid or solid medium.

The term "heterologous" in reference to a polynucleotide or protein refers to a polynucleotide or protein that does not naturally occur in a host cell. In some embodiments, the protein is a commercially important industrial protein. It is intended that the term encompass proteins that are encoded by naturally occurring genes, mutated genes, and/or synthetic genes. The term "homologous" in reference to a polynucleotide or protein refers to a polynucleotide or protein that occurs naturally in the host cell.

The term "introduced" in the context of inserting a nucleic acid sequence into a cell includes "transfection," "transforniation," or "transduction" and refers to the incorporation of a nucleic acid sequence into a eukaryotic or prokaryotic cell wherein the nucleic acid sequence may be incorporated into the genome of the cell (*e.g.*, chromosome, plasmid, plastid, or mitochondrial DNA), converted into an autonomous replicon, or transiently expressed.

As used herein, the terms "transformer," "stably transformed," and "transgenic" refer to a cell that has a nonnative (*e.g.*, heterologous) nucleic acid sequence integrated into its genome or as an episomal plasmid that is maintained through multiple generations.

The terms "isolated," and "separated" as used herein refer to a material (*e.g.,* a protein, nucleic acid, or cell) that is removed from at least one component with which it is naturally associated. For example, these terms may refer to a material which is substantially or essentially free from components which normally accompany it as found in its native state, such as, for example, an intact biological system.

The term "recovered" as used herein refers to at least partial separation of an enzyme from one or more soluble components of a microbial broth and/or at least partial separation from one or more solvents in the broth, e.g., water or ethanol. A recovered enzyme is often of higher purity than prior to the recovery process. However, in some embodiments, a recovered enzyme may be of the same or lower purity than prior to the recovery process.

The term "secreted protein" refers to a region of a polypeptide that is released from a cell. In some embodiments, the secreted protein is the protein that is released or cleaved from a recombinant fusion polypeptide.

The term "secretion" refers to the selective movement of a protein across a membrane in a host cell to the extracellular space and surrounding media.

As used herein the term "specific activity" means an enzyme unit defined as the number of moles of substrate converted to product by an enzyme preparation per unit time under specific conditions. Specific activity is expressed as units (U)/mg of protein.

As used herein, the terms "detergent composition" and "detergent formulation" are used in reference to mixtures which are intended for use in a wash medium for the cleaning of soiled objects. In some preferred embodiments, the term is used in reference to laundering fabrics and/or garments (*e.g.*, "laundry detergents"). In alternative embodiments, the term refers to other detergents, such as those used to clean dishes, cutlery, etc. (*e.g.*, "dishwashing detergents"). It is not intended that the present invention be limited to any particular detergent formulation or composition. Indeed, it is intended that in addition to enzyme, the term encompasses detergents that contain surfactants, transferase(s), hydrolytic enzymes, oxido reductases, builders, bleaching agents, bleach activators, bluing agents and fluorescent dyes, caking inhibitors, masking agents, enzyme activators, antioxidants, and solubilizers.

As used herein, the term "disinfecting" refers to the removal of contaminants from the surfaces, as well as the inhibition or killing of microbes on the surfaces of items. It is not intended that the present invention be limited to any particular surface, item, or contaminant(s) or microbes to be removed.

"Microbial broth" or "fermentation, broth" refers to a growth medium in which microbial (*e.g.*, bacterial or fungal) cells are grown and which is suitable for expression of an enzyme as described herein.

An "insoluble enzyme" refers to an enzyme that is present in a solid phase. An insoluble enzyme separates (*i.e.*, partitions) with a solid phase upon separation of solid and liquid phases, for example, solid and liquid phases of a microbial broth. It is understood that in a whole microbial broth some enzyme will typically also be found in the soluble phase in the medium in addition to the insoluble enzyme that is part of the solid phase. The insoluble enzyme may be bound to solids in the microbial broth, such as cell solids or other solid components or may be precipitated or crystallized within the microbial broth.

"Solubilization" or "solubilize" as used herein refers to removal of conditions or substances that cause precipitation of an enzyme or keep the enzyme in an insoluble form, or changing of physical conditions (for example, change in temperature; mixing; sonication) or addition of solubilization substances (for example, solubilization solvents, *e.g.*, water; polyols; salts; acids; bases; surfactants) that render the insoluble enzyme soluble.

"purifying" or "purification of" an insoluble enzyme refers to refinement of an enzyme of interest by partially or fully removing components that are not of interest, for example, liquid components of a microbial broth that are not of interest. A purified enzyme-containing composition contains a lower amount of one or more components than the mixture from which the enzyme was purified.

Enzyme "purity" in a sample may be calculated by dividing enzyme activity by total amount of dry solids. Dry solids is a measure of all the materials present after liquid has been removed. Dry solids can include, *inter alia,* protein, salt, carbohydrate, metabolites, nuclei acid, lipids, cells, cell debris, and spent fermentation media. Enzyme purity can be expressed as a ratio in relation to one or more of the components of the dry solids.

"Cell debris" refers to cell walls and other insoluble cellular components that are released after disruption of the cell membrane, *i.e.*, after lysis of microbial cells.

Broth conditioning" refers to pretreatment of a microbial fermentation broth designed to improve subsequent broth handling properties. Broth conditioning changes the chemical composition and/or physical and/or rheological properties of the broth to facilitate its use in downstream recovery and/or formulation processes. Broth conditioning may include one or more treatments such as pH modification, heat treatment, cooling, addition of additives (*e.g.*, calcium, salt(s), flocculant(s), reducing agent(s), enzyme activator(s), enzyme inhibitor), and/or surfactant(s)), mixing, and/or timed hold (*e.g.*,.0.5 to 200 hours) of the broth without further treatment.

"ATCC" refers to American Type Culture Collection located at Manassas, Va. 20108 (www.atcc.org).

"NRRL" refers to the Agricultural Research Service Culture Collection, National Center for Agricultural Utilization Research (and previously known as USDA Northern Regional Research Laboratory), Peoria, Ill.

"A," "an" and "the" include plural references unless the context clearly dictates otherwise.

### *Recovery methods*

The invention provides a method including a step of recovering an insoluble enzyme from a microbial broth (*i.e.,* a microbial fermentation medium in which cells that express the enzyme have been grown), without removal of cells or cell debris from the broth. Recovery of the insoluble enzyme includes separating the insoluble enzyme partially or substantially completely from soluble components of the microbial broth, *i.e.,* separating the insoluble enzyme from at least a portion of the soluble components of the microbial broth. The recovery processes described herein convert fermentation broth containing one or more insoluble enzymes of interest into recovered enzyme(s) of interest that may be formulated for use without removal of cells or cell debris.

In one embodiment, the method includes providing a microbial broth in which microbial cells that express the enzyme have been grown and in which at least some of the enzyme is insoluble, and recovering the insoluble enzyme without removing the cells, thereby producing a composition that includes recovered insoluble enzyme and microbial cells and/or cell debris. In another embodiment, the expressed enzyme is soluble in the microbial broth and at least some of the enzyme is rendered insoluble by addition of one or more precipitant(s). Microbial cells may be lysed before or after recovery of the insoluble enzyme, producing insoluble cell debris.

In some embodiments, a microbial broth containing an expressed soluble or insoluble enzyme is combined with a composition containing an externally produced soluble or insoluble enzyme. One or more precipitant(s) is added to render at least one of the enzymes (the enzyme expressed by the microbial cells that produced the microbial broth and/or the enzyme that was added to the microbial broth) insoluble. The method comprises recovering enzyme expressed by the microbial cells in insoluble form.

In some embodiments, soluble enzyme may be co-recovered with insoluble enzyme when at least part of the liquid phase is removed from the microbial broth.

Generally, the recovered insoluble enzyme is present as an inclusion body.

In some embodiments, two or more microbial broths from separate microbial fermentations are combined, and one or more insoluble enzyme is recovered from the combined microbial broths.

In methods of the invention, the insoluble enzyme is enzymatically active, *i.e.,* capable of catalyzing an enzymatic reaction. The insoluble enzyme has catalytic potential, *i.e.,* the enzyme is catalytically active in insoluble form and/or after solubilization in a solvent and under conditions suitable for catalytic activity to occur. In one embodiment, the insoluble enzyme is catalytically active in both insoluble fonn and after solubilization. In one embodiment, the insoluble enzyme is inactive in insoluble form and becomes catalytically active after solubilization.

In various embodiments, any of at least about 90, 80, 70, 60, 50, 40, 30, 20, or 10% of the enzyme is insoluble in the microbial broth prior to recovery of the insoluble enzyme. In some embodiments, the purity of the insoluble enzyme is increased by at least about 10% after recovery of the insoluble enzyme

Recovery of insoluble enzyme in microbial broth containing microbial cells and/or cell debris may include, for example, concentration and/or diafiltration. The microbial broth containing insoluble enzyme may be concentrated to remove at least part of the soluble portion of the broth, thereby increasing the concentration of the insoluble enzyme in the broth, by one or more techniques that are well known in the art, including but not limited to membrane processes (*e.g.*, ultrafiltration, microfiltration, nanofiltration), centrifugation, and evaporation. Alternatively, or in conjunction with concentration, diafiltration may be employed to replace at least part of the soluble portion of the microbial broth, typically by using a membrane process to diafilter the microbial broth against water.

In methods of the invention, microbial cells are grown in a growth medium under conditions suitable for expression of an enzyme of interest, thereby producing a microbial broth containing the enzyme of interest. For example, the enzyme of interest may be expressed in a host cell from an expression vector. The enzyme of interest may be secreted into the growth medium or may be expressed intracellularly and not secreted.

An enzyme that is secreted into the growth medium may be naturally insoluble in the growth medium under the conditions used for growth of the cells, or insolubility of the enzyme may be induced by adding a precipitant prior to recovery of the enzyme. Examples of precipitants include, but are not limited to salts (*e.g.,* NaCl, NaSO$_4$, ammonium sulfate, magnesium sulfate, concentration about 0.1 to about 50% (w/v)), polyelectrolytes (*e.g.*, alginate, carboxymethycellulose, polyacrylic acid, tannic acid, polyphosphates), and alteration in temperature or pH, or a combination thereof. (See, *e.g.,* Isolation and Purification of Proteins (2003) R. Hatti-Kaul and B. Mattiasson, ed., Marcel Dekker, Inc., pp. 225-275; Bioseparations: Science and Engineering (2003) R.G. Harrison, P. Todd, S.R. Rudge, D.P. Petrides, pp. 243-271; Protein Purification: Principles and Practice (1994) R.K. Scopes, Springer, pp. 71-101.; Protein Purification Process Engineering (1994) R.G. Harrison, ed., Marcel Dekker Inc., "Differential Precipitation of Proteins," pp. 115 -208.)

Soluble enzymes may also be rendered insoluble by affinity interactions, e.g., substrate-enzyme interactions or inhibitor-enzyme interactions, for example, by contact with an affinity resin.

An enzyme that is expressed intracellularly and not secreted may be released by lysis of the cells. The released enzyme may be naturally insoluble under conditions in the growth medium into which it is released, or insolubility may be induced by addition of a precipitant prior to recovery of the enzyme as described above.

Microbial cells which may be used to express an enzyme of interest as described herein may be eukaryotic or prokaryotic, for example, a fungal or bacterial microorganism. Examples of eukaryotic microorganisms that may be used include, but are not limited to, *Trichoderma* sp., *Hypocrea* sp., *Aspergillus* sp., *Penicillium* sp., *Saccharomyces* sp., *Schizosaccharomyces* sp., *Hansenula* sp., and *Fusarium* sp. Examples of prokaryotic microorganisms that may be used include, but are not limited to, *Bacillus* sp., *Streptomyces* sp., *Pantoea* sp., *Escherichia* sp., and *Pseudomonas* sp.

Any enzyme that is expressed in an insoluble form or which is expressed in soluble form and can be rendered insoluble, for example by addition of a precipitant, may be recovered in accordance with the methods described herein, and formulated in a granular formulation as described herein. In some embodiments, the enzyme is a hydrolase, for example, a protease (bacterial (*e.g.,* a subtilisin) or fungal; acid, neutral, or alkaline), an amylase (alpha or beta), a lipase, or a cellulase. In some embodiments, the enzyme is a subtilisin, for example, as described in U.S. Patent No. 4,760,025, EP Patent No. 130 756, or PCT Application No. WO 91106637: In some embodiments, the enzyme is an alpha-amylase as described in PCT Application No. WO08/112459. In some embodiments, the enzyme is a cellulase, for examples, Multifect L250™ or Puradax™, commercially available from Genencor, Division of Danisco US, Inc. In some embodiments, the enzyme is an oxidase, an oxygenase, a transferase, a dehydratase, a reductase, a hemicellulase, a peroxidase, a phospholipase, an esterase, a cutinase, a pectinase, a keratinase, a lipoxygenase, a ligninase, a pullanase, a tannase, a pentosanase, a malanase, a β-glucanase, an arabinosidase, a hyaluronidase, a chondroitinase, a laccase, a catalase, an isomerase, a pectate lyase, or a mannanase, or a combination thereof. In some embodiments, the enzyme is a phytase. In some embodiments, the enzyme is a perhydrolase enzyme, such as, for example, an enzyme as described in PCT Application No. WO 05/056782.

In recovery methods described herein, a composition that includes insoluble enzyme and microbial cells and/or cell debris is produced. In some embodiments, the composition includes intact microbial cells. In one embodiment, the intact microbial cells are live microbial cells. In one embodiment, the intact microbial cells are dead microbial cells. (See, *e.g.,* U.S. Patent No. 5,801,034, which describes a method for killing cells without lysis by adjusting the pH of a fermentation mixture to a value equal to or less than about two pH units below the pK$_a$ of the compatible organic acid using a mineral acid, and adding a sufficient amount of a compatible organic acid and/or organic acid salt to the mixture to effect a substantially complete cell kill.)

In some embodiments, the composition includes lysed microbial cells, *i.e.,* cell debris produced by lysis of the microbial cells. In one embodiment, the method includes disrupting microbial cell membranes to produce a microbial cell lysate prior to recovery of the insoluble protein. In the case of an enzyme that is soluble in the microbial broth and for which insolubility is induced by addition of one or more precipitant, disruption of the microbial cell membranes may occur either before or after addition of the precipitant(s). In some embodiments, disruption of the cell membranes is achieved by contacting the cells with an enzyme that is capable of microbial cell lysis, for example, a lysozyme enzyme. In one embodiment, cells that have been lysed with an enzyme that is capable of microbial cell lysis are further homogenized to produce a homogenized microbial cell lysate. In other embodiments, disruption of the cell membranes is achieved by mechanical homogenization.

An enzyme-containing composition comprising one or more insoluble enzymes and microbial cells and/or cell debris, prepared as described herein, is formulated in a granular formulation.

*Granular formulations*

Low dusting, stable enzyme granules may be produced from microbial broth containing insoluble enzyme and microbial cells and/or cell debris. The microbial broth containing insoluble enzyme and microbial cells and/or cell debris is produced in accordance with any of the recovery methods described above. Enzyme granules may be produced by methods that are well known in the art, for example, in a top-spray fluid bed coater, in a bottom spray Wurster coater, or by drum granulation (*e.g.,* high shear granulation) extrusion, or pan coating.

The invention provides a method of making an enzyme-containing granule, comprising providing a composition that contains insoluble enzyme and microbial cells and/or cell debris, produced via a recovery method as described above, and producing an enzyme-containing granule that includes the composition, wherein the insoluble enzyme in the granule is enzymatically active, *i.e.,* capable of enzymatic activity when contacted by a substrate for the enzyme under conditions that are suitable for catalytic activity to occur. The enzyme is catalytically active in insoluble form and/or when solubilized in a solvent, in the presence of substrate and under conditions suitable for enzymatic catalysis to occur, for example, in an application of use for the enzyme-containing granules. In one embodiment, the enzyme is catalytically active in both insoluble form and after solubilization. In one embodiment, the enzyme is catalytically inactive in insoluble form and becomes catalytically active after solubilization.

In one embodiment, the enzyme-containing granule is produced in a top-spray fluid bed processor. In one embodiment, an enzyme-containing layer that includes the composition containing insoluble enzyme and microbial cells and/or cell debris, as described above, is coated onto a core in the top-spray fluid bed processor. Optionally, the granule may contain a salt layer between the core and the enzyme-containing layer. The salt may be selected from, for example, sodium sulfate, sodium citrate, magnesium sulfate, potassium sulfate, and ammonium sulfate, or a mixture thereof. One or more further layers may be coated over the enzyme-containing layer. For example, a barrier salt layer may be coated over the enzyme-containing layer. In some embodiments, one or more outer coating layers may be coated over the enzyme-containing layer, or over a barrier salt layer that is coated over the enzyme-containing layer. Outer coating layer(s) may contain, for example, polymer(s) and/or pigment(s).

In some embodiments, a composition comprising an insoluble enzyme and microbial cells and/or cell debris, as described above, is incorporated into a core, and one more layers are coated over the enzyme-containing core. For example, a barrier salt layer may be coated over the enzyme-containing core. In some embodiments, one or more outer coating layers may be coated over the enzyme-containing core, or over a barrier salt layer that is coated over the enzyme-containing core. Outer coating layer(s) may contain, for example, polymer(s) and/or pigment(s).

In some embodiments, a granule containing an insoluble enzyme and microbial cells and/or cell debris is produced by drum granulation, such as high shear granulation. Such a granule contains an enzyme-containing core (containing the insoluble enzyme and microbial cells and/or cell debris) and one or more coating layers over the core. Such coating layers may include, but are not limited to, a polymer, *e.g.,* polyethylene glycol or polyvinyl alcohol, a pigment such as titanium dioxide, or glycerol.

The granules may also include further components such as, but not limited to plasticizers, fillers, and lubricants.

Described herein are enzyme-containing granules comprising a core and an enzyme-containing composition in a layer surrounding the core or incorporated into the core, and optionally one or more additional layers. The enzyme-containing composition contains an insoluble enzyme and microbial cells and/or microbial cell debris produced as described above. The insoluble enzyme is enzymatically active in the granule, *i.e.,* capable of enzymatic activity when contacted by a substrate for the enzyme under conditions that are suitable for catalytic activity to occur.

The granules exhibit low dust, for example, less than 50, 40, 30, 20, 10, 5, 4, 2, 1, or 0.5 mg/pad, as measured by the Heubach attrition test. The granules are stable when stored under ambient humidity and temperature conditions, but soluble or dispersible upon contact with water so as to release the enzyme when in use:

Small granules without protective coatings generate a significant amount of durst during pneumatic transport and filling operations, both in the enzyme granule manufacturing plant and at the customer's manufacturing plant. Dust can be both a hygienic problem and a manufacturing problem, so it must be minimized as much as possible. Several industrial tests have been developed to measure the mechanical resistance to attrition and dusting formation of different granular enzyme formulations. These include the Heubach attrition test and the elutriation test. The Heubach test subjects particles to defined crushing and fluidization forces by using rotating paddles to roll steel balls through a bed of granules contained within cylindrical chamber and simultaneously percolating a stream of air through the bed to strip off any dust that is generated. The generated dust is drawn by vacuum through a tube and deposited onto a filter pad outside the Heubach chamber. The weight or active components of the dust collected is referred to as Heubach dust. In the elutriation test, granules are placed on a glass frit within a tall glass tube and fluidized with a constant dry airstream over a fixed period of time. A discussion of the principles, operation and limitations of the Heubach and elutriation dust tests can be found for example, in "Enzymes In Detergency" ed. Jan H. van Ee., Ch. 15, pgs. 310 - 312, (Marcel Dekker, Inc. New York (1997) and references cited therein. The Heubach dust test is also described in U.S. Patent Nos. 5,324,649, 5,879,920, and 7,108,821.

*Core*

The core is the inner nucleus of the granule. Suitable cores for use in the present invention are preferably of a highly hydratable material (i.e., a material which is readily dispersible or soluble in water). The core material should either disperse in water (disintegrate when hydrated) or solubilize in water by going into a true aqueous solution. Clays (bentonite, kaolin), nonpareils and agglomerated potato starch are considered dispersible. Nonpareils are spherical particles consisting of a seed crystal that has been built onto and rounded into a spherical shape by binding layers of powder and solute to the seed crystal in a coating apparatus. Nonpareils are typically made from a combination of a sugar such as sucrose, and a powder such as cornstarch. Suitable cores further include seed crystal materials include sucrose crystals, sodium chloride or sodium sulfate seeds, and other inorganic salts which may be built up with ammonium sulfate, sodium sulfate, potassium sulfate and the like.

Granules composed of inorganic salts and/or sugars and/or small organic molecules may be used as the cores of the present invention. Suitable water soluble ingredients for incorporation into cores include: sodium chloride, ammonium sulfate, sodium sulfate, urea, citric acid, sucrose, lactose and the like. Water-soluble ingredients can be combined with water dispersible ingredients. Cores for use in the granules may further comprise one or more of the following: active ingredients, polymers, fillers, plasticizers, fibrous materials, extenders and other compounds known to be used in cores. Suitable polymers include - polyvinyl alcohol (PVA), polyethylene glycol, polyethylene oxide, and polyvinyl pyrrolidine. The PVA may be partially hydrolyzed (70 - 90%); intermediately hydrolyzed (90 - 98%); fully hydrolyzed (98-99%); super hydrolyzed (99 - 100%) PVA, or a mixture thereof, with a low to high degree of viscosity.

The core of an enzyme-containing granule as described herein may consist of one or more inorganic salts or a sucrose crystal. In some embodiments, the core consists of sodium sulfate, sodium citrate, sodium chloride, calcium sulfate, or a combination thereof. In one embodiment, the core consists of sodium sulfate.

Suitable fillers useful in the cores include inert materials used to add bulk and reduce cost, or used for the purpose of adjusting the intended enzyme activity in the finished granule. Examples of such fillers include, but are not limited to, water soluble agents such as urea, salts, sugars and water dispersible agents such as clays, talc, silicates, carboxymethyl cellulose and starches.

Suitable plasticizers useful in the cores are nonvolatile solvents added to a polymer to reduce its glass transition temperature, thereby reducing brittleness and enhancing deformability. Typically, plasticizers are low molecular weight organic compounds and are highly specific to the polymer being plasticized. Examples include, but are not limited to, sugars (such as, glucose, fructose and sucrose), sugar alcohols (such as, sorbitol, xylitol and maltitol) polyols (polyhydric alcohols for example, alcohols with many hydroxyl radical groups such as glycerol, ethylene glycol, propylene glycol or polyethylene glycol), polar low molecular weight organic compounds, such as urea, or other known plasticizers such as dibutyl or dimethyl phthalate, or water.

Suitable fibrous materials useful in the cores of the present invention include materials which have high tensile strength and which can be formed into fine filaments having a diameter of 1 to 50 microns and a length equal to at least four diameters. Typical fibrous materials include, but are not limited to: cellulose, glass fibers, metal fibers, rubber fibers, azlon (manufactured from naturally occurring proteins in corn, peanuts and milk) and synthetic polymer fibers. Synthetics include Rayon®, Nylon®, acrylic, polyester, olefin, Saran®, Spandex® and Vinal®. Typically cellulose fibers have an average fiber length of 160 microns with a diameter of about 30 microns.

Cores can be fabricated by a variety of granulation techniques well known in the art including: crystallization, precipitation, pan-coating, fluid-bed coating, rotary atomization, extrusion, spheronization, drum granulation and high-shear agglomeration.

In one embodiment of the present invention, the core is a water-soluble or dispersible nonpareil (either sugar or salt as described above) which optionally can be further coated by or built up from the seed crystal (nonpareil) using polyvinylalcohol (PVA) either alone or in combination with anti-agglomeration agents such as titanium dioxide, talc, or plasticizers such as sucrose or polyols. The level of PVA in the coating of the nonpareil may represent from about 0.5% to 20% of the weight of the coated nonpareil.

In general, the core including any active ingredient incorporated therein is an impact-sensitive particle. However, the invention is not limited by the type of core, and numerous patents and publications describe cores that may be used in the invention and reference is made to USP 5,879,920; USP 4,689,287 and WO 00/24877.

*Enzymes*

One or more insoluble enzymes may be used in the low-dusting, stable enzyme granules described herein. An enzyme-containing composition comprising one or more insoluble enzymes and microbial cells and/or cell debris, prepared as described above, is incorporated into the granules described herein.

In some embodiments, the enzyme is a hydrolase, for example, a protease (bacterial *(e.g.*, a subtilisin) or fungal; acid, neutral, or alkaline), an amylase (alpha or beta), a lipase, or a cellulase. In some embodiments, the enzyme is a subtilisin, for example, as described in U.S. Patent No. 4,760,025, EP Patent No. 130 756, or PCT Application No. WO 91/06637. In some embodiments, the enzyme is an alpha-amylase as described in PCT Application No. WO08/112459. In some embodiments, the enzyme is a cellulase, for example, Multifect L250™ or Puradax™, commercially available from Genencor, Division of Danisco US, Inc. In some embodiments, the enzyme is an oxidase, an oxygenase, a transferase, a dehydratase, a reductase, a hemicellulase, a peroxidase, a phospholipase, an esterase, a cutinase, a pectinase, a keratinase, a lipoxygenase, a ligninase, a pullulanase, a tannase, a pentosanase, a malanase, a β-glucanase, an arabinosidase, a hyaluronidase, a chondroitinase, a laccase, a catalase, an isomerase, a pectate lyase, or a mannanase, or a combination thereof. In some embodiments, the enzyme is a phytase. In some embodiments, the enzyme is a perhydrotase enzyme, such as, for example, an enzyme as described in PCT Application No. WO 05/056782. In some embodiments, the enzyme is capable of hydrolyzing a substrate *(e.g.*, a stain).

In one aspect, one or more insoluble enzymes in a composition that contains microbial cells and/or cell debris are incorporated in the core of the granule. In another preferred aspect one or more enzymes in a composition that contains microbial cells and/or cell debris are layered around a core. When layered around the core, the layer comprising the enzyme may additionally include a binder such as a polymer, for example a vinyl polymer such as polyvinyl alcohol (PVA).

A layer comprising the enzyme may further optionally comprise one or more other components in addition to the enzyme-containing composition. Such non-enzyme components include, but are not limited to, polymers *(e.g.*, polyvinyl alcohol, polyethylene glycol), sugars *(e.g.*, sucrose, saccharose, glucose, fructose, galactose, maltodextrin), starches *(e.g.*, corn starch, wheat starch, tapioca starch, potato starch, chemically or physically modified starch), dextrins, antifoam agents *(e.g.*, polyether polyols such as Foamblast 882 (Emerald Foam Control), Erol DF 204K (Ouvrie PMC), DG436 (ODG Industries, Inc.), KFO 880 (KABO Chemicals, Inc.)), sugar alcohols *(e.g.*, sorbitol, maltitol, lactitol, xylitol), surfactants *(e.g.*, alcohol ethoxylates such as Neodol 23-6.5 (Shell Chemical LP, Houston, TX) and Lutensol TO65 (BASF)), and anti-redeposition agents *(e.g.*, polyethylene glycol polyesters such as Repel-o-Tex SRP6 (Rhodia, Inc.), Texcare SRN-100 or SRN-170 (Clariant GmbH, Sorez-100(ISP Corp.)).

An "antifoam agent" is a compound that is used to prevent or break foam. These can also be referred to as defoamers, or defoaming agents. These compounds are surface active substances which decrease the surface elasticity of liquids and prevent metastable foam formation. The foam breaks as a result of the tendency to attain the equilibrium between the surface elasticity of the liquid and the surface active substances. (Vardar-Sukan (1991) Recent Adv. Biotechnol. 113-146). Antifoams useful in the granules described herein are generally suitable for use in a bioprocess. Suitable antifoam agents include, but are not limited to, fats, oils, waxes, aliphatic acids or esters, alcohols, sulfates, sulfonates, fatty acids, soaps, nitrogenous compounds, phosphates, polyglycols, sulfides, thio compounds, siloxanes and halogenated and inorganic compounds. (Ghildyal (1988) Adv. Appl. Microbiol. (1988) 33:173-222). In some embodiments, oils, fatty acids, esters, polyglycols and siloxanes are useful. In some embodiments, the antifoam agent is ethylene oxide propylene oxide copolymer. In one embodiment, the oxide propylene oxide copolymer has an approximate molecule weight of 2200 *(e.g.*, available as Mazu™ from Mazer Chemicals, Inc.).

The granules may include between 0.01% to 50% or 75% by weight of enzyme solids (recovered insoluble enzyme and microbial cells and/or cell debris, produced as described above). In various embodiments, a granule may comprise any of at least about 0.5, 5,10, 20, 30 or 40% enzyme solids by weight. A layer comprising the enzyme solids, including any binders and other components therein, may comprise between about 0.2% to 70% or 80% by weight of the granule.

In some embodiments, an enzyme-containing layer having a viscosity less than 20 centipoise facilitates agglomeration free granulation.

*Coating Layers*

The granules may further comprise one or more other coating layers. Coating layers may serve any of a number of functions depending on the end use of the granule. For example, coatings may render the active ingredient, particularly enzymes, resistant to oxidation by bleach, or coating layers may bring about the desirable rate of dissolution upon introduction of the granule into an aqueous medium, or provide a further barrier against ambient moisture in order to enhance the storage stability of the granule and reduce the possibility of microbial growth within the granule.

The coating layer may comprise one or more polymer(s) and, optionally, a low residue pigment or other excipients such as lubricants. Such excipients are known to those skilled in the art. Furthermore, coating agents may be used in conjunction with other active agents of the same or different categories.

Suitable polymers include PVA and/or PVP or mixtures of both. If PVA is used, it may be partially hydrolyzed, fully hydrolyzed or intermediately hydrolyzed PVA having low to high degrees of viscosity (preferably partially hydrolyzed PVA having low viscosity). Other vinyl polymers which may be useful include polyvinyl acetate and polyvinyl pyrrolidone. Useful copolymers include, for example, PVA-methylmethacrylate copolymer. Other polymers such as PEG may also be used in the outer layer. These further coating layers may further comprise one or more of the following: plasticizers, pigments, lubricants such as surfactants or antistatic agents and, optionally, additional enzymes. Suitable plasticizers useful in the coating layers of the present invention are those disclosed herein above. Suitable pigments useful in the coating layers of the present invention include, but are not limited to, finely divided whiteners such as titanium dioxide or calcium carbonate, or colored pigments, or a combination thereof. Preferably such pigments are low residue pigments upon dissolution.

As used herein "lubricants" mean any agent which reduces surface friction, lubricates the surface of the granule, decreases static electricity or reduces friability of the granules. Lubricants can also play a related role in improving the coating process, by reducing the tackiness of binders in the coating. Thus, lubricants can serve as anti-agglomeration agents and wetting agents.

Suitable lubricating agents include, but are not limited to, surfactants (ionic, nonionic or anionic), fatty acids, antistatic agents and antidust agents. Preferably the lubricant is a surfactant, and most preferably is an alcohol-based surfactant such as a linear, primary alcohol of a 9 to 15 carbon atom chain length alkane or alkene or an ethoxylate or elhoxysulfate derivative thereof. Such surfactants are commercially available as the Neodol® product line from Shell International Petroleum Company. Other suitable lubricants include, but are not limited to, antistatic agents such as StaticGuard™, Downey™, Triton X100 or 120 and the like, antidust agents such as Teflon™ and the like, or other lubricants known to those skilled in the art.

Other intermediate layers, such as binders, structuring agents, and barrier layers may be included. Suitable harrier materials include, for examples, inorganic salts, sugars, or organic acids or salts. Structuring agents can be polysaccharides or polypeptides. Preferred structuring agents include starch, modified starch, carrageenan, cellulose, modified cellulose, gum arabic, guar gum, acacia gum, xanthan gum, locust bean gum, chitosan, gelatin, collagen, casein, polyaspartic acid and polyglutamic acid. Preferably, the structuring agent has low allergenicity. A combination of two or more structuring agents can be used in the granules. Binders include but are not limited to sugars and sugar alcohols. Suitable sugars include but are not limited to sucrose, glucose, fructose, raffinose, trehalose, lactose and maltose. Suitable sugar alcohols include sorbitol, mannitol and inositol.

*Barrier layer*

In some embodiments, a barrier layer is coated over the enzyme layer or over an enzyme-containing core in an enzyme-containing granule as described herein. In some embodiments, the barrier layer contains one or more salts, for example, sodium sulfate, sodium citrate, magnesium sulfate, potassium sulfate, and/or ammonium sulfate. In some embodiments, the barrier layer comprises, consists of, or consists essentially of sodium sulfate. In some embodiments, the barrier layer contains a sugar (*e.g.,* sucrose), a polysaccharide (*e.g.,* starch), or a combination thereof.

In some embodiments, the barrier layer is hydrated. The term "hydrated" means that the barrier material contains water in a free or bound form, or a combination of the two. The water of hydration can be added either during or after the coating process. The degree of hydration will be a function of the material itself and the temperature, humidity and drying conditions under which it is applied.

"Moderate or high" water activity means having a water activity of at least about 0.25, 0.30, or 0.35. The water activity referred to herein is that of the granule itself once it has the barrier material-but no further coatings--coated onto it. Further coatings may mask accurate measurement of the water activity of the barrier material as a distinct layer.

Without wishing to be bound by theory, it is expected that materials with a water activity greater than 0.25 will have a reduced driving force for picking up water under storage conditions in which the relative humidity is greater than 25%. Most climates have relative humidities above 25%. Many detergents have water activities in the range of about 0.3 to 0.4. If the water activity of the granule is actually higher than that of the surrounding detergent or storage climate, the driving force for pick up of water by the granule should be eliminated, and in fact water may be given up by the granule to its surroundings. Even if the water activity of the granule is lower than that of the detergent or the corresponding relative humidity, the water present in the barrier layer would act as a shield limiting the amount of water being picked up by the granule and affecting the protein core.

In the case of salt hydrates, the hydrated material is a crystalline salt hydrate with bound waters of crystallization. The hydrate should be chosen and applied in a manner such that the resulting coated granule will have a water activity in excess of 0.25, or as high as possible while still retaining granule which is dry to the touch. By applying a salt hydrate, or any other suitable hydrated barrier material, in such a manner, one eliminates any driving force for further uptake of water by the granule. As an important consequence, the driving force for transport of substances which may be detrimental to enzyme activity, such as perborate or peroxide anion, is removed. Without water as a vehicle, these substances are less likely to penetrate the enzyme core. Empirical data demonstrates that enzyme activity in the granule is substantially enhanced by coating the enzyme core with stable salt hydrates.

Examples of suitable salts for production of a hydrated barrier layer include magnesium sulfate heptahydrate, zinc sulfate heptahydrate, copper sulfate pentahydrate, sodium phosphate dibasic heptahydrate, magnesium nitrate hexahydrate, sodium borate decahydrate, sodium citrate dihydrate and magnesium acetate tetrahydrate.

## Outer coating layer

In some embodiments, an enzyme containing granule comprises an outer coating layer. In one embodiment, the outer coating layer is coated over the enzyme layer. In some embodiments, an outer coating layer is coated over one or more intermediate coating layers. In one embodiment, the outer coating layer is coated over a barrier layer, which is coated over the enzyme layer or over an enzyme-containing core.

In some embodiments, the outer coating layer includes one or more polymers. Suitable polymers include, but are not limited to, polyvinyl alcohol (IPVA), polyvinyl pyrrolidone (PVP), polyvinyl acetate, PVA-methylmethacrylate copolymer, PVP-PVA copolymer, cellulose derivatives such as methylcellulose, hydroxypropylmelhyl cellulose, hydroxycellulose, ethylcellulose, carboxymethyl cellulose, hydroxypropyl cellulose, polyethylene glycol, polyethylene oxide, chitosan, gum arabic, xanthan, carrageenan, latex polymers, and enteric polymer.

In some embodiments, the outer coating layer includes PVA. Suitable PVAs for incorporation in the outer coating layer include partially hydrolyzed, fully hydrolyzed, and intermediately hydrolyzed PVAs having low to high degrees of viscosity. (See, *e.g.,* U.S. Patent No. 5,324,649.) In one embodiment, the outer coating layer includes partially hydrolyzed PVA having low viscosity.

In some embodiments, the outer coating layer includes one or more pigments. Nonlimiting examples of suitable pigments include finely divided whiteners, such as titanium dioxide or calcium carbonate, calcium sulfate, talc, or colored pigments or dyes. Typically, such pigments are low residue pigments upon dissolution. In addition to polymers and/or pigments, the outer coating layer may also include one or more of plasticizers, extenders, lubricants, surfactants, and anti-redeposition agents.

Suitable plasticizers include, but are not limited to, polyols (*e.g.*, sugars, sugar alcohols, polyethylene glycols (PEGs), glycol, propylene glycol), urea, triethyl citrate, dibutyl or dimethyl phthalate, or water.

Suitable extenders include, but are not limited to, sugars (*e.g.*, sucrose or starch hydrolysates, such as maltodextrin or corn syrup solids), clays (*e.g.,* kaolin or bentonite), and talc. An "extender" is a substance (generally lower cost) added to another substance (generally higher cost and higher performance) to modify or dilute it.

Suitable lubricants include, but are not limited to, nonionic surfactants (*e.g.*, Neodol, Lutensol TO 65), tallow alcohols, fatty acids, fatty acid salts (*e.g.,* magnesium stearate), and fatty acid esters.

Suitable surfactants include, but are not limited to, alcohol ethoxylates such as Neodol 23-6.5 and Lutensol TO65.

Suitable anti-redeposition agents include, but are not limited to, polyethylene glycol polyesters such as Repel-o-Tex SRP6, Texcare SRN-100 or SRN-170, and Sorex-100.

## Other Adjunct ingredients

Adjunct ingredients may be added to the granules, including but not limited to: metallic salts, solubilizers, activators, antioxidants, dyes, inhibitors, binders, fragrances, enzyme protecting agents/scavengers such as ammonium sulfate, ammonium citrate, urea, guanidine hydrochloride, guanidine carbonate, guanidine sulfonate, thiourea dioxide, monethyanolamine, diethanolamine, triethanolamine, amino acids such as glycine, sodium glutamate and the like, proteins such as bovine serum albumin, casein and the like, etc., surfactants, including anionic surfactants, ampholytic surfactants, nonionic surfactants, cationic surfactants and long-chain fatty acid salts, builders, alkalis or inorganic electrolytes, bleaching agents, bluing agents and fluorescent dyes, and caking inhibitors. Surfactants are described, for example, in PCT Application PCT/US92/00384.

*Matrix Granules*

In one embodiment, the granule includes a protein "matrix" which includes the composition containing the insoluble enzyme and microbial cells and/or cell debris mixed together with a combination of a sugar or sugar alcohol and a structuring agent (See, *e.g.,* EP1037968B1.) Optionally, a barrier layer can be layered over the enzyme-containing matrix or a barrier material can be included in the enzyme-containing matrix. Also, optionally, a coating can be applied over a seed particle, an enzyme matrix surrounding the seed particle, and/or the barrier layer. In some embodiments, the structuring agent is a polysaccharide or a polypeptide. The matrix can be homogenous throughout the core or can be layered over a seed particle. There can be one or more layers between the seed particle and the matrix or the matrix and the barrier layer, for example, a coating such as polyvinyl alcohol (PVA). A matrix granule can be produced, for example, in a top-spray fluid bed processor.

Seed particles are inert particles upon which the enzyme matrix can be layered can be composed of inorganic salts, sugars, sugar alcohols, small organic molecules such as organic acids or salts, minerals such as clays or silicates or a combination of two or more of these. Suitable soluble ingredients for incorporation into seed particles include: sodium chloride, potassium chloride, ammonium sulfate, sodium sulfate, sodium sesquicarbonate, urea, citric acid, citrate, sorbitol, mannitol, oleate, sucrose, lactose and the like. Soluble ingredients can be combined with dispersible ingredients such as talc, kaolin or bentonite. Seed particles can be fabricated by a variety of granulation techniques including: crystallization, precipitation, pan-coating, fluid-bed coating, fluid-bed agglomeration, rotary atomization, extrusion, prilling, spheronization, drum granulation and high shear agglomeration. In the granules described herein, if a seed particle is used then the ratio of seed particles to granules is 1:1.

Suitable sugars include sugars such as sucrose, glucose, fructose, raffinose, trehalose, lactose and maltose. Suitable sugar alcohols include sorbitol, mannitol and inositol. The ratio of sugar or sugar alcohol to structuring agent in the matrix is preferably 0.1-90% by weight of the protein matrix. The sugar or sugar alcohol in the matrix can be sugar or sugar alcohol added to the protein or can be from the fermentation broth in which the protein is present.

The structuring agent can be a polysaccharide or a polypeptide. These classes of compounds have the simultaneous desirable properties of high molecular weight and high water solubility. Without wishing to be bound by theory, it is believed that the high molecular weight of the structuring agent contributes two important properties which a sugar or sugar alcohol matrix alone would lack: (1) providing cohesion and strength to the particle, greatly reducing the tendency of the particle to dust; and (2) serving as a diffusion barrier to water and small molecules by virtue of forming a polymer network or "cage" throughout the matrix structure. This greatly improves the stability of the granule.

Preferred structuring agents include starch, modified starch, carrageenan, cellulose, modified cellulose, gum arabic, guar gum, acacia gum, xanthan gum, locust bean gum, chitosan, gelatin, collagen, casein, polyaspartic acid and polyglutamic acid. Preferably, the structuring agent has low allergenicity. A combination of two or more structuring agents can be used in the granules.

The enzyme-containing matrix may further contain one or more synthetic polymers or other excipients as known to those skilled in the art. Suitable synthetic polymers include polyethylene oxide, polyvinyl alcohol, polyvinyl pyrrolidone, polyethylene glycol and polyethylene oxide/polypropylene oxide. The matrix may also further contain plasticizers and anti-agglomeration agents. Suitable plasticizers useful in the present invention include polyols such as glycerol, propylene glycol, polyethylene glycol (PEG), urea, or other known plasticizers such as triethyl citrate, dibutyl or dimethyl phthalate or water. Suitable anti-agglomeration agents include fine insoluble or sparingly soluble materials such as talc, $TiO_2$, clays, amorphous silica, magnesium stearate, stearic acid and calcium carbonate.

The matrix granules can further contain a barrier layer. A barrier layer is used to slow or prevent the diffusion of substances that can adversely affect the protein or enzyme into the matrix. The barrier layer is made up of a barrier material and can be coated over the protein core or the barrier material can be included in the protein core. Suitable barrier materials include, for example, inorganic salts or organic acids or salts. The matrix without the protein can also be used as a barrier layer.

The matrix granules of can also contain one or more coating layers. For example, such coating layers may be one or more intermediate coating layers or such coating layers may be one or more outside coating layers or a combination thereof. Coating layers may serve any of a number of functions in a granule composition, depending on the end use of the enzyme granule. For example, coatings may render the enzyme resistant to oxidation by bleach, bring about the desirable rates of dissolution upon introduction of the granule into an aqueous medium, or provide a barrier against ambient moisture in order to enhance the storage stability of the enzyme and reduce the possibility of microbial growth within the granule.

Suitable coatings include water soluble or water dispersible film-forming polymers such as polyvinyl alcohol (PVA), polyvinyl pyrrolidone (PVP), cellulose derivatives such as methylcellulose, hydroxypropyl methylcellulose, hydroxycellulose, ethylcellulose, carboxymethyl cellulose, hydroxypropyl cellulose, polyethylene glycol, polyethylene oxide, gum arabic, xanthan, carrageenan, chitosan, latex polymers, and enteric coatings. Furthermore, coating agents may be used in conjunction with other active agents of the same or different categories.

Suitable PVAs for incorporation in the coating layer(s) of the granule include partially hydrolyzed, fully hydrolyzed and intermediately hydrolyzed PVAs having low to high degrees of viscosity. Preferably, the outer coating layer comprises partially hydrolyzed PVA having low viscosity. Other vinyl polymers which may be useful include polyvinyl acetate and polyvinyl pyrrolidone. Useful copolymers include, for example, PVA-methylmethacrylate copolymer and PVP-PVA copolymer. The coating layers of may further contain one or more of the following: plasticizers, extenders, lubricants, pigments, and optionally additional enzymes. Suitable plasticizers useful in the coating layers of the present invention are plasticizers including, for example, polyols such as sugars, sugar alcohols, or polyethylene glycols (PEGs), urea, glycol, propylene glycol or other known plasticizers such as triethyl citrate, dibutyl or dimethyl phthalate or water. Suitable pigments useful in the coating layers of the present invention include, but are not limited to, finely divided whiteners such as titanium dioxide or calcium carbonate or colored pigments and dyes or a combination thereof. Preferably such pigments are low residue pigments upon dissolution. Suitable extenders include sugars such as sucrose or starch hydrolysates such as maltodextrin and corn syrup solids, clays such as kaolin and bentonite and talc. Suitable lubricants include nonionic surfactants such as Neodol, tallow alcohols, fatty acids, fatty acid salts such as magnesium stearate and fatty acid esters.

## *Compositions*

Described herein are compositions containing enzyme-containing granules as described above. In addition to the enzyme-containing granules, the compositions contain components suitable for use of the granules in particular applications of use, such as cleaning (*e.g.,* detergents), textile processing, animal feed, brewing, baking, food, or beverage applications..

### *Cleaning compositions*

In some embodiments, enzyme-containing granules as described herein are incorporated into a cleaning composition, such as a detergent, e.g., for laundry or dishwashing use, to provide cleaning performance and/or cleaning benefits. Enzymes suitable for inclusion in a cleaning composition include, but are not limited to, hemicellulases, peroxidases, proteases, cellulases, xylanases, lipases; phospholipases, esterases, cutinases, pectinases, keratinases, reductases, oxidases, phenoloxidases, lipoxygenases, ligninases, pullulanases, tannases, pentosanases, malanases, β-glucanases, arabinosidases, hyaluronidase, chondroitinase, laccases, perhydrolases, and amylases, or mixtures thereof. A typical combination is a cocktail of conventional applicable enzymes like protease, lipase, cutinase and/or cellulase in conjunction with amylase.

Adjunct materials may also be included in the cleaning composition, for example, to assist or enhance cleaning performance, for treatment of the substrate to be cleaned, or to modify the aesthetics of the cleaning composition as is the case with perfumes, colorants, dyes or the like. It is understood that such adjuncts are in addition to the enzyme-containing granules as described herein. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, deposition aids, dispersants, enzyme stabilizers, catalytic materials, bleach activators, bleach boosters, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are described in U.S. Patent Nos. 5,576,282, 6,306,812, and 6,326,348.

In some embodiments, the cleaning composition does not contain phosphate. In some embodiments, the cleaning composition is a non-phosphate-containing dishwashing detergent. In some embodiments, the cleaning composition is a non-phosphate-containing laundry detergent. In some embodiments, the non-phosphate cleaning composition contains a non-phosphate builder, e.g., citrate.

Surfactants - A cleaning composition as described herein may comprise a surfactant or surfactant system wherein the surfactant can be selected from nonionic surfactants, anionic surfactants, cationic surfactants, ampholytic surfactants, zwitterionic surfactants, semi-polar nonionic surfactants, and mixtures thereof. A surfactant is typically present at a level of about 0.1 % to about 60%, about 1 % to about 50% or about 5% to about 40% by weight of the subject cleaning composition.

Builders - A cleaning composition as described herein may comprise one or more detergent builder or builder system. When a builder is used, the subject cleaning composition will typically comprise at least about 1%, about 3% to about 60%, or about 5% to about 40% builder by weight of the subject cleaning composition.

Builders include, but are not limited to, the alkali metal, ammonium and alkanolammonium salts of polyphosphates, alkali metal silicates, alkaline earth and alkali metal carbonates, aluminosilicate builders, polycarboxylate compounds. ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacelic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof.

Chelating Agents - A cleaning composition as described herein may contain one or more chelating agent. Suitable chelating agents include, but are not limited to, copper, iron and/or manganese chelating agents and mixtures thereof. When a chelating agent is used, the cleaning composition may comprise about 0.1 % to about 15%, or about 3.0% to about 10% chelating agent by weight of the subject cleaning composition.

Deposition Aid - A cleaning composition as described herein may contain a one or more deposition aid. Suitable deposition aids include, but are not limited to, polyethylene glycol, polypropylene glycol, polycarboxylate, soil release polymers such as polytelephthalic acid, and clays such as Kaolinite, montmorillonite, atapulgite, illite, bentonite, halloysite, and mixtures thereof.

Dye Transfer Inhibiting Agents - A cleaning composition as described herein may include one or more dye transfer inhibiting agent. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones, and polyvinylimidazoles, and mixtures thereof. When present in a subject cleaning composition, dye transfer inhibiting agent may be present at levels of about 0.0001% to about 10%, about 0.01% to about 5%, or about 0.1% to about 3% by weight of the cleaning composition.

Dispersants - A cleaning composition as described herein may contain one or more dispersant. Suitable water-soluble organic dispersants include, but are not limited to, the homo-or co-polymeric acids or their salts, in which the polycarboxylic acid comprises at least two carboxyl radicals separated from each other by not more than two carbon atoms.

Enzyme Stabilizers - Enzymes for use in detergents can be stabilized by various techniques. Enzymes employed herein can be stabilized, for example, by the presence of water-soluble sources of calcium and/or magnesium ions in the finished compositions that provide such ions to the enzymes.

Catalytic Metal Complexes - A cleaning composition as described herein may include one or more catalytic metal complex. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, elhylenediaminetetra (methylenephosphonic acid) and water-soluble salts thereof. Such catalysts are disclosed in U.S. Patent No. 4,430,243. Manganese-containing catalysts useful herein are known, and are described, for example, in U.S. Patent No. 5,576,282. Cobalt bleach catalysts useful herein are known, and are described, for example, in U.S. Patent Nos. 5,597,936 and 5,595,967. Such cobalt catalysts are readily prepared by known procedures, such as taught for example in U.S. Patent Nos. 5,597,936 and U.S. 5,595,967.

Compositions herein may also include a transition metal complex of a macropolycyclic rigid ligand - abreviated as "MRL". As a practical matter, and not by way of limitation, the compositions and cleaning processes herein can be adjusted to provide on the order of at least one part per hundred million of the active MRL species in the aqueous washing medium, and will often provide about 0.005 ppm to about 25 ppm, about 0.05 ppm to about 10 ppm, or about 0.1 ppm to about 5 ppm, of the MRL in the wash liquor. Suitable transition-metals in a transition-metal bleach catalyst include manganese, iron and chromium. In one embodiment, an MRL is an ultra-rigid ligand that is cross-bridged, such as 5, 12-diethyl- 1,5,8,12-tetraazabicyclo[6.6.2] hexadecane. Suitable transition metal MRLs are readily prepared by known procedures, such as taught for example in PCT Application No. WO 00/332601 and U.S. Patent No. 6,225,464.

The cleaning compositions disclosed herein can be used to clean a situs on a surface or fabric. Typically at least a portion of the situs is contacted with a cleaning composition as described above, in neat form or diluted in a wash liquor, and then the situs is optionally washed and/or rinsed. Washing includes, but is not limited to, scrubbing, and mechanical agitation. A fabric may comprise most any fabric capable of being laundered in normal consumer use conditions. The disclosed cleaning compositions are typically employed at concentrations of from about 500 ppm to about 15,000 ppm in solution. When the wash solvent is water, the water temperature typically ranges from about 5 °C to about 90 °C and, when the situs comprises a fabric, the water to fabric mass ratio is typically from about 1:1 to about 30:1.

*Textile processing compositions*

In some embodiments, enzyme-containing granules as described herein are incorporated into a textile processing composition. Enzymes suitable for inclusion in a textile processing composition include, but are not limited to, cellulases, perhydrolases, polyesterases, amylases, catalases, pectinases, and laccases. In some embodiments, a textile processing composition may also include an anti-redeposition agent (*e.g.,* Repel-O-Tex, Sorez 100 (ISP Corp.).

*Animal feed compositions*

In some embodiments, enzyme-containing granules as described herein are incorporated into an animal feed composition. Enzymes suitable for inclusion in a feed composition include cellulolytic and/or hemicellulolytic enzymes. Nonlimiting examples of enzymes suitable for incorporation into a feed composition include phytases, xylanases, phosphatases, proteases, amylases, esterases, redox enzymes, lipases, transferases, cellulases, phospholipases, ligninases, and β-glucanases.

The following examples are intended to illustrate, but not limit, the invention.

## EXAMPLES

### Example 1

### Preparation of Enzyme Concentrate and Granular Formulation from Fermentation Broth Containing Partially Precipitated Enzyme using Conventional Recovery Process

### *Preparation of Bacterial Lysate*

*Bacillus licheniformis* that recombinantly expresses α-amylase enzyme as described in PCT Application No. WO08/112459 was fermented using well-known techniques in the art. After fermentation, the broth contained precipitated enzyme. A sample of the broth was centrifuged at 10,000 rpm for 5 min. The resulting supernatant contained 10% of the total enzyme activity measured in the whole broth before centrifugation. 90% of the enzyme was insoluble. Alpha-amylase activity was determined as described in U.S. Patent Application No. 12/041,917.

For cell lysis, the broth temperature was adjusted to 40°C and 0.01% (w/w) lysozyme was added. The broth was held for 48 hrs, and then diluted with 0.6 parts water and immediately flocculated with 4% (w/w) cationic polymer (C581, from Cytec Industries, Inc.). Diatomaceous earth (FW12, from World Minerals) was added at 4% (w/w) to the flocculated broth and filtered through a dead end vacuum filter fitted with HR9000 filter pad coated with FW12 diatomaceous cake. The enzyme yield in the filtrate after cell separation was 6%.

The clarified filtrate was concentrated with an ultrafilter fitted with a 10K molecular weight cutoff (MWCO) polyethersulfone (PES) spiral wound membrane (KOCH Membrane Systems). The final concentrate contained 11.5 g/l or 1.15% (w/v) active α-amylase and 27.04% (w/w) dry solid matter.

The overall yield for this process was 5% of the starting enzyme activity in whole broth and the purity was 4.3% (active dry solids/total dry solids).

### *Granular Formulation*

A granular composition was prepared from the enzyme concentrate as follows.

**Spray 1:** 513 grams of sodium sulfate crystal (Saltec), with a particle diameter size range of 150 μm to 350 μm, was loaded into a Vector Fl-1 fluid bed coater and fluidized. To this, 2062 grams of solution containing 11.5 g/L of active amylase, 1.6% talc (Nytal 400), 0.5% polyvinyl alcohol (Erkol 5/88), 1.0% anti-foam (Foamblast 882 from Emerald Performance Materials), and 1.6% corn starch (Cargill Foods) was spray-coated onto the sodium sulfate crystals. The spray coating parameters were as follows:

| | |
|---|---|
| Solution Spray Rate | 3.6 gpm (grams per minute), increasing to 13.6 gpm over 1.5 hours. Kept at 13.6 gpm for remainder of experiment. |
| Inlet Temperature | 69°C, increased to 72°C after 30 minutes |
| Outlet Temperature | Between 43.9°C and 46.2°C |
| Fluidization Air Flow | Between 76.0 cfm (cubic feet per minute) and 81.4 cfm |
| Atomization Air Pressure | 30 psi (pounds per square inch), increasing to 40 psi over 1.5 hours. |

1043 grams of product was harvested.

**Spray 2:** 1043 grams of the enzyme granules from spray 1 were loaded into a Vector FL-1 fluid bed coater and fluidized. 1667 grams of an aqueous solution containing 333 grams of sodium sulfate (20 % of granule composition) were then spray coated onto the enzyme granules.

The spray coating parameters were as follows:

| | |
|---|---|
| Solution Spray Rate | 8.3 gpm (grams per minute), increasing to 19.2 gpm over 15 minutes. Kept at 19.2 gpm for remainder of experiment. |
| Inlet Temperature | 63°C, increased to 80°C after 15 minutes |
| Outlet Temperature | Between 44.2°C and 47.8°C |
| Fluidization Air Flow | Between 79.5 cfm (cubic feet per minute) and 82.6 cfm |
| Atomization Air Pressure | 40 psi |

1363 grams of product was harvested.

**Spray 3:** 1363 grams of the enzyme granules from spray 2 were loaded into a Vector FL-1 fluid bed coater and fluidized. 1065 grams of an aqueous solution containing 83 grams (5% of granule composition) of poly vinyl alcohol (5/88 from Erkol), 83 grams (5% of granule composition) of titanium dioxide (R902 from DuPont), and 25 grams (1.5% of granule composition) of Neodol (23-6.5 from Shell Chemicals) were then sprayed coated onto the enzyme granules. The spray coating parameters were as follows:

| | |
|---|---|
| Solution Spray Rate | 4.2 gpm (grams per minute), increasing to 12.2 gpm over 30 minutes. Kept at 12.2 gpm for remainder of experiment. |
| Inlet Temperature | 75°C, increased to 78°C after 30 minutes |
| Outlet Temperature | Between 46.0°C and 52.4°C |
| Fluidization Air Flow | Between 78.4 cfm (cubic feet per minute) and 81.2 cfm |
| Atomization Air Pressure | 40 psi |

1487 grams of final product was harvested.

Using the Heubach attrition test, the granules were analyzed for dust. The dust level of the granules was 1.66 mg/pad.

### Example 2

**Preparation of Enzyme Concentrate and Granular Formulation from Fermentation Broth Containing Partially Precipitated Enzyme by Dissolving the Precipitate Followed by Conventional Recovery Process**

#### *Preparation of Enzyme Concentrate*

*Bacillus licheniformis* that recombinantly expresses α-amylase enzyme as described in PCT Application No. WO08/112459 was fermented using well-known techniques in the art. After fermentation, the broth contained precipitated enzyme. A sample of the broth was centrifuged at 10,000 rpm for 5 min. The resulting supernatant contained 5% of the total enzyme activity measured in the whole broth before centrifugation. 95% of the enzyme was insoluble.

For cell lysis, the broth temperature was adjusted to 40°C and 0.01% (w/w) lysozyme was added. The broth was held for 1.5 hrs. The temperature was then increased to 60°C and held at 60°C for 1 hr. The broth was then diluted with 1 part water.

The precipitated enzyme remained insoluble after the heat step and the dilution. The lysate was then incubated with gentle mixing at room temperature for 28 hours with 5% (w/w) glucose and 3% (w/w) NaCl at pH 9 before cell separation. Most of the precipitated α-amylase activity (84%) was solubilized.

The lysate was then flocculated with 4% (w/w) cationic polymer (C581, from Cytec Industries, Inc.). Diatomaceous earth (FW12, from World Minerals) was added at 4% (w/w) to the flocculated broth and filtered through a rotary vacuum drum filter pre-coated with FW12 diatomaceous cake. The enzyme yield in the filtrate after cell separation was 87%.

The clarified filtrate was concentrated with an ultrafilter fitted with a 10K MWCO PES spiral wound membrane (KOCH Membrane Systems). The final concentrate contained 60 g/l or 6% (w/v) active α-amylase and 27% (w/w) dry solid matter.

The overall yield for this process was 84% of the starting enzyme activity in whole broth and the purity was 22% (active dry solids/total dry solids). However, the concentrate contained glucose and NaCl. Removal of these substances would adversely impact the cost of the process.

*Granular Formulation*

A granular composition was prepared from the enzyme concentrate as follows.

**Spray 1:** 478 grams of sucrose crystal, with a particle diameter size range of 200μm to 600μm, was loaded into a Vector F1-1 fluid bed coater and fluidized. To this, 628 grams of solution containing 60.0 g/L of active amylase, 115 grams (9.8% of granule composition) of corn starch (Cargill Foods) was spray-coated onto the sucrose crystals. The spray coating parameters were as follows:

| | |
|---|---|
| Solution Spray Rate | 2.9 gpm (grams per minute), increasing to 17.3 gpm over 52 minutes. Kept at 20.2 gpm for remainder of experiment. |
| Inlet Temperature | Between 71 °C and 74 °C for spray 1 |
| Outlet Temperature | Between 44.7°C and 48.5°C |
| Fluidization Air Flop | Between 83.5 cfm (cubic feet per minute) and 87.2 cfm |
| Atomization Air Pressure | 32 psi (pounds per square inch), increasing to 40 psi over 52 minutes. |

700 grams of product was harvested.

**Spray 2:** 700 grams of the enzyme granules from spray 1 were loaded into a Vector FL-1 fluid bed coater and fluidized. 1308 grams of an aqueous solution containing 120 grams (10.2% of granule composition) of sucrose (C&H Sugars), 120 grams (10.2% of granule composition) of corn starch (Cargill Foods), 72 grams (6.1% of granule composition) of titanium dioxide (R902 DuPont), and 15 grams (1.3% of granule composition) of Neodol (23-6.5 from Shell Chemicals) were then spray coated onto the enzyme granules.

The spray coating parameters were as follows:

| | |
|---|---|
| Solution Spray Rate | 12.2 gpm (grams per minute), increasing to 20.2 gpm over 10 minutes. Kept at 20.2 gpm for remainder of experiment. |
| Inlet Temperature | 74°C, increased to 82°C after 15 minutes |
| Outlet Temperature | Between 46.3°C and 47.7°C |
| Fluidization Air Flow | Between 85.0 cfm (cubic feet per minute) and 88.0 cfm |
| Atomization Air Pressure | Between 35 psi and 36 psi |

988 grams of product was harvested.

**Spray 3:** 988 grams of the enzyme granules from spray 2 were loaded into a Vector FL-1 fluid bed coater and fluidized. 586 grams of an aqueous solution containing 29 grams (2.5% of granule composition) of Hydroxy propyl methyl cellulose (HPMC E-15 Dow Chemicals), and 4 grams (0.3% of granule composition) of polyethylene glycol 600 (Carbowax by Electron Microscopy Sciences) were then sprayed coated onto the enzyme granules.

The spray coating parameters were as follows:

| | |
|---|---|
| Solution Spray Rate | 8.1 gpm (grams per minute), increasing to 15.6 gpm over 45 minutes. Kept at 15.6 gpm for remainder of experiment. |
| Inlet Temperature | Between 75 °C and 82 °C |
| Outlet Temperature | Between 43.6°C and 50.3°C |
| Fluidization Air Flow | Between 85.0 cfm (cubic feet per minute) and 87.8 cfm |
| Atomization Air Pressure | 40 psi |

1014 grams of final product was harvested.

Using the Heubach attrition test, the granules were analyzed for dust. The dust level of the granules was 2.80 mg/pad.

### Example 3

**Preparation of Enzyme Concentrate and Granular Formulation from Homogenized Cell Lysate from Fermentation Broth Containing Partially Precipitated Enzyme**

*Reparation of Homogenized Cell Lysate*

*Bacillns licheniformis* that recombinantly expresses $\alpha$-amylase enzyme as described in PCT Application No. WO08/112459 was fermented using well-known techniques in the art. After fermentation, the broth contained precipitated enzyme. A sample of the broth was centrifuged at 10,000 rpm for 5 min. The resulting supernatant contained 6% of the total enzyme activity measured in the whole broth before centrifugation.

Cell lysis was performed as described in Example 2. The lysate was then chilled to 10°C and homogenized at 6,000 psi in a Gaulin Homogenizer. The enzyme yield was 90%, and the purity was 6% (active dry solids/total dry solids).

*Granular Formulation*

A granular composition was prepared from the homogenized lysate as follows.

**Spray 1:** 17.64 kilograms of sodium sulfate crystal (Saltec), with a particle diameter size range of 150 $\mu$m to 350 $\mu$m, was loaded into a Deseret 60 fluid bed coater and fluidized. To this, 259.41 kilograms of solution containing 12.53 g/L of active amylase, 5.88 kilograms (5% of granule composition) talc (Nytal 400), 1.18 kilograms (1.0% of granule composition) anti-foam (Foamblast 882 from Emerald Performance Materials), 7.06 kilograms (6% of granule composition) corn starch (Cargill Foods), and 1.18 kilograms (1.0% of granule composition) Neodol (23-6.5 from Shell Chemicals) was spray-coated onto the sodium sulfate crystals. The spray coating parameters were as follows:

| | |
|---|---|
| Solution Spray Rate | 50 gpm (grams per minute), increasing to 500 gpm over 3 hours. Kept at 500 gpm for remainder of experiment. |
| Inlet Temperature | Between 73°C and 85°C |
| Outlet Temperature | Between 42.7°C and 55.7°C |
| Fluidization Air Flow | Between 800 cfm (cubic feet per minute) and 952 cfm |
| Atomization Air Pressure | 60 psi (pounds per square inch), increasing to 68 psi over 5 hours. |

65.2 kilograms of product was harvested.

**Spray 2:** 65.2 kilograms of the enzyme granules from spray 1 were loaded into a Deseret 60 fluid bed coater and fluidized. 111.11 kilograms of an aqueous solution containing 22.22 kilograms of sodium sulfate (20 % of granule composition) were then spray coated onto the enzyme granules. The spray coating parameters were as follows:

| | |
|---|---|
| Solution Spray Rate | Between 500 gpm (grams per minute) to 580 gpm over entire spray 2. |
| Inlet Temperature | 85°C |
| Outlet Temperature | Between 48.3°C and 54.1°C |
| Fluidization Air Flow | Between 1109 cfm (cubic feet per minute) and 1234 cfm |
| Atomization Air Pressure | 70 psi |

88 kilograms of product was harvested.

**Spray 3:** 84.967 kilograms of the enzyme granules from spray 2 were loaded into a Deseret 60 fluid bed coater and fluidized. 84.97 kilograms of an aqueous solution containing 6.12 kilograms (5.2% of granule composition) of poly vinyl alcohol (5/88 from Erkol), 6.12 kilograms (5.2% of granule composition) of titanium dioxide (R902 from DuPont), 1.53 kilograms (1.3% of granule composition) of talc (Nytal 400) and 1.53 kilograms (1.3% of granule composition) of Neodol (23-6.5 from Shell Chemicals) were then sprayed coated onto the enzyme granules. The spray coating parameters were as follows:

| | |
|---|---|
| Solution Spray Rate | Between 310 gpm (grams per minute) to 400 gpm over entire spray 3.. |
| Inlet Temperature | 85°C |
| Outlet Temperature | Between 48.8°C and 56.8°C |
| Fluidization Air Flow | Between 1070 cfm (cubic feet per minute) and 1238 cfm |
| Atomization Air Pressure | Between 70 psi and 76 psi |

97.6 kilograms of product was harvested.

**Spray** 4: 97.6 kilograms of the enzyme granules from spray 3 were loaded into a Deseret 60 fluid bed coater and fluidized. 9.41 kilograms of an aqueous solution containing 471 grams (0.4% of granule composition) of Neodol (23-6.5 from Shell Chemicals) were then sprayed coated onto the enzyme granules.

The spray coating parameters were as follows:

| | |
|---|---|
| Solution Spray Rate | 295 gpm over entire spray 4 |
| Inlet Temperature | 85°C |
| Outlet Temperature | Between 47.1°C and 51.1°C |
| Fluidization Air Flow | Between 1126 cfm (cubic feet per minute) and 1220 cfm |
| Atomization Air Pressure | Between 65 psi and 68 psi |

101.2 kilograms of product was harvested.

Using the Heubach attrition test, the granules were analyzed for dust. The dust level of the granules was 0.33 mg/pad.

## Example 4

### Preparation of Enzyme Concentrate and Granular Formulation from Homogenized and Diafiltered Cell Lysate from Fermentation Broth Containing Partially Precipitated Enzyme

### *Preparation of Homogenized Cell Lysate*

*Bacillus licheniformis* that recombinantly expresses $\alpha$-amylase enzyme as described in PCT Application No. WO08/112459 was fermented using well-known techniques in the art. After fermentation, the broth contained precipitated enzyme. A homogenized lysate was prepared as described in Example 3.

The homogenized lysate was diafiltered and concentrated with an ultrafilter fitted with a 10K MWCO PES spiral wound membrane (KOCH Membrane Systems). The final concentrate contained 11 g/l or 1.1% (w/v) active $\alpha$-amylase and 16.7% (w/w) dry solid matter.

The homogenized lysate was diafiltered using city water. Constant volume diafiltration was performed, *i.e.,* by holding the volume in the feed tank containing the homogenized lysate constant, adding city water to replace the volume of permeate removed from the system. The total amount of diafiltration water used was 0.5x of the initial homogenized lysate. The diafiltered homogenized lysate was further concentrated to 16% (w/w) dry solids. The purity of the resulting preparation was 9.3% (active dry solids/total dry solids). The purity was 1.25 times higher than the homogenized lysate prepared in Example 3.

### *Granular Formulation*

A granular composition was prepared from the homogenized and diafiltered lysate as follows.

**Spray 1:** 29.606 kilograms of sodium sulfate crystal (Saltec), with a particle diameter size range of 150 $\mu$m to 350 $\mu$m, was loaded into a Deseret 60 fluid bed coater and fluidized. To this, 170.79 kilograms of solution containing 14.2 g/L of active amylase, 5.88 kilograms (5% of granule composition) talc (Nytal 400), 1.18 kilograms (1.0% of granule composition) anti-foam (Foamblast 882 from Emerald Performance Materials), 11.77 kilograms (10% of granule composition) corn starch (Cargill Foods), and 1.18 kilograms (1.0% of granule composition) Neodol (23-6.5 from Shell Chemicals) was spray-coated onto the sodium sulfate crystals. The spray coating parameters were as follows:

| | |
|---|---|
| Solution Spray Rate | 106 gpm (grams per minute), increasing to 550 gpm over 5 hours. Kept at 550 gpm for remainder of experiment. |
| Inlet Temperature | Between 80°C and 82.6°C |
| Outlet Temperature | Between 51.0°C and 60.9°C |
| Fluidization Air Flow | Between 804 cfm (cubic feet per minute) and 1058 cfm |
| Atomization Air Pressure | 65 psi (pounds per square inch), increasing to 70 psi over 5 hours |

66.6 kilograms of product was harvested.

**Spray 2:** 66.6 kilograms of the enzyme granules from spray 1 were loaded into a Deseret 60 fluid bed coater and fluidized. 111.11 kilograms of an aqueous solution containing 22.22 kilograms of sodium sulfate (20 % of granule composition) were then spray coated onto the enzyme granules. The spray coating parameters were as follows:

| | |
|---|---|
| Solution Spray Rate | Between 400 gpm (grams per minute) to 640 gpm over entire spray 2. |
| Inlet Temperature | Between 83.5°C and 85.6 °C |
| Outlet Temperature | Between 51.4°C and 54.8°C |
| Fluidization Air Flow | Between 1097 cfm (cubic feet per minute) and 1246 cfm |
| Atomization Air Pressure | 70 psi |

93.8 kilograms of product was harvested.

**Spray 3:** 93.8 kilograms of the enzyme granules from spray 2 were loaded into a Deseret 60 fluid bed coater and fluidized. 84.97 kilograms of an aqueous solution containing 6.12 kilograms (5.2% of granule composition) of poly vinyl alcohol (5/88 from Erkol), 6.12 kilograms (5.2% of granule composition) of titanium dioxide (R902 from DuPont), 1.53 kilograms (1.3% of granule composition) of talc (Nytal 400) and 1.53 kilograms (1.3% of granule composition) of Neodol (23-6.5 from Shell Chemicals) were then sprayed coated onto the enzyme granules. The spray coating parameters were as follows:

| | |
|---|---|
| Solution Spray Rate | Between 200 gpm (grams per minute) to 410 gpm over entire spray 3. |
| Inlet Temperature | Between 79.6°C and 83.9 °C |
| Outlet Temperature | Between 55.9°C and 59.6°C |
| Fluidization Air Flow | Between 1121 cfm (cubic feet per minute) and 1244 cfm |
| Atomization Air Pressure | Between 70 psi and 75 psi |

108.4 kilograms of product was harvested.

**Spray 4:** 108.4 kilograms of the enzyme granules from spray 3 were loaded into a Deseret 60 fluid bed coater and fluidized. 9.41 kilograms of an aqueous solution containing 471 grams (0.4% of granule composition) of Neodol (23-6.5 from Shell Chemicals) were then sprayed coated onto the enzyme granules. The spray coating parameters were as follows:

| | |
|---|---|
| Solution Spray Rate | 250 gpm over entire spray 4 |
| Inlet Temperature | Between 82.7°C and 82.9 °C |
| Outlet Temperature | Between 57.4°C and 59.7°C |
| Fluidization Air Flow | Between 1157 cfm (cubic feet per minute) and 1299 cfm |
| Atomization Air Pressure | 70 psi |

110.0 kilograms of product was harvested.

Using the Heubach attrition test, the granules were analyzed for dust. The dust level of the granules was 0.15 mg/pad.

## Example 5

## Preparation of Enzyme Concentrate and Granular Formulation from Diafiltered Cell Lysate from Fermentation Broth Containing Partially Precipitated Enzyme

### *Preparation of Diafiltered Cell Lysate*

*Bacillus licheniformis* that recombinantly expresses α-amylase enzyme as described in PCT Application No. WO08/112459 was fermented using well-known techniques in the art. After fermentation, the broth contained precipitated enzyme. The cells were lysed as described in Example 2. The lysate was diafiltered using city water as described in Example 4. The diafiltered lysate was further concentrated to 18.2% (w/w) dry solids. The purity of the resulting preparation was 17% (active dry solids/total dry solids). The purity was 1.7 times higher than the homogenized lysate prepared in Example 3.

**Example 6**

**Preparation of Enzyme Concentrate and Granular Formulation from Diafiltered Cell Lysate from Fermentation Broth Containing: Partially Precipitated Enzyme**

*Preparation of Diafiltered Cell Lysate*

*Bacillus licheniformis* that expresses $\alpha$-amylase enzyme as described in PCT Application No. WO08/11245 was fermented as described in Example 1. The lysate was diafiltered and concentrated as in Example 5. The resulting preparation had an enzyme activity of 2.14% and the dry solids were 17.2%.

*Granular Formulation*

A granular composition was prepared from the diafiltered lysate as follows.

**Spray 1**: 691 grams of sodium sulfate crystal (Saltec), with a particle diameter size range of 150 $\mu$m to 350 $\mu$m, was loaded into a Vector Fl-1 fluid bed coater and fluidized. To this, 1470 grams of solution containing 23.41 g/L of active amylase, 5% talc (Nytal 400), 1.0% anti-foam (Foamblast 882 from Emerald Performance Materials), and 5% corn starch (Cargill Foods) was spray-coated onto the sodium sulfate crystals. The spray coating parameters were as follows:

| | |
|---|---|
| Solution Spray Rate | 2.6 gpm (grams per minute), increasing to 14.1 gpm over 1.5 hours. |
| Inlet Temperature | 69°C, increased to 72°C after 30 minutes |
| Outlet Temperature | Between 44.9°C and 45.8°C |
| Fluidization Air Flow | Between 78.6 cfm (cubic feet per minute) and 80.8 cfm |
| Atomization Air Pressure | 30 psi (pounds per square inch), increasing to 40 psi over 1.5 hours. |

1050 grams of product was harvested.

**Spray 2:** 1050 grams of the enzyme granules from spray 1 were loaded into a Vector FL-1 fluid bed coater and fluidized. 1667 grams of an aqueous solution containing 333 grams of sodium sulfate (20 % of granule composition) were then spray coated onto the enzyme granules. The spray coating parameters were as follows:

| | |
|---|---|
| Solution Spray Rate | Between 15.6 gpm (grams per minute) and 17.4 gpm |
| Inlet Temperature | Between 78°C and 82°C |
| Outlet Temperature | Between 46.0°C and 47.5°C |
| Fluidization Air Flow | Between 78.6 cfm (cubic feet per minute) and 82.0 cfm |
| Atomization Air Pressure | 40 psi |

1352 grams of product was harvested.

**Spray 3:** 1352 grams of the enzyme granules from spray 2 were loaded into a Vector FL-1 fluid bed coater and fluidized. 1127 grams of an aqueous solution containing 88 grams (5% of granule composition) of poly vinyl alcohol (5/88 from Erkol), 88 grams (5% of granule composition) of titanium dioxide (R902 from DuPont), and 26 grams (1.5% of granule composition) of Neodol (23-6.5 from Shell Chemicals) were then sprayed coated onto the enzyme granules. The spray coating parameters were as follows:

| | |
|---|---|
| Solution Spray Rate | 3.2 gpm (grams per minute), increasing to 10.2 gpm over 60 minutes. Kept at 10.2 gpm for remainder of experiment. |
| Inlet Temperature | 75°C, increased to 80C after 10 minutes |
| Outlet Temperature | Between 52.0°C and 53.7°C |
| Fluidization Air Flow | Between 82.5 cfm (cubic feet per minute) and 83.1 cfm |
| Atomization Air Pressure | 40 psi |

1535 grams of final product was harvested.

Using the Heubach attrition test, the granules were analyzed for dust. The dust level of the granules was 1.50 mg/pad.

### Example 7

**Preparation of Bacterial Broth Concentrate with Soluble Enzyme and Granular Formulation**

*Preparation of Enzyme Concentrate*

*Bacillus subtilis* that expresses subtilisin protease was fermented using well-known techniques in the art. After fermentation, the broth contained mostly soluble subtilisin enzyme. The broth was centrifuged at 10,000 rpm for 5 min. The resulting supernatant contained activity that was the same as the activity in a whole broth sample. The broth was adjusted to pH 5.8±0.2, diluted with 1.5±0.2 parts of city water and immediately flocculated with 8% C311 (Kemira). The flocculated broth was filtered through a rotary vacuum drum filter precoated with diatomaceous earth (FW12, from World Minerals). The clarified filtrate was concentrated with an ultrafilter fitted with a 10K MWCO PES spiral wound membrane (KOCH Membrane Systems). The final concentrate contained 57.23 g/L and 19.1% (w/w) dry solid matter. The subtilisin activity in the enzyme concentrate remained soluble before granulation, as determined by the supernatant (14,000 rpm, 4 min) activity of the enzyme concentrate, which was the same the same activity as the whole enzyme concentrate.

*Granular Formulation*

A granular composition was prepared from the ultrafiltration concentrate as follows.

**Spray 1:** 643 grams of sodium sulfate crystal (Saltec), with a particle diameter size range of 150 $\mu$m to 350 $\mu$m, was loaded into a Vector Fl-1 fluid bed coater and fluidized. To this, 593 grams of solution containing an active protease, 0.7% talc (Nytal 400), 0.9% anti-foam (Foamblast 882 from Emerald Performance Materials), and 0.5% of poly vinyl alcohol (5/88 from Erkol), was spray-coated onto the sodium sulfate crystals. The spray coating parameters were as follows:

| | |
|---|---|
| Solution Spray Rate | 3.5 gpm (grams per minute), increasing to 9.0 gpm over 1.5 hours. |
| Inlet Temperature | 57°C, increased to 86°C after 1 hour 15 minutes |
| Outlet Temperature | Between 45.2°C and 48.9°C |
| Fluidization Air Flow | Between 72.4 cfm (cubic feet per minute) and 83.6 cfm |
| Atomization Air Pressure | 30 psi (pounds per square inch), increasing to 37 psi over 30 minutes. |

769 grams of product was harvested.

**Spray 2:** 769 grams of the enzyme granules from spray 1 were loaded into a Vector FL-1 fluid bed coater and fluidized. 2001 grams of an aqueous solution containing 400 grams of sodium sulfate (27.7 % of granule composition) were then spray coated onto the enzyme granules. The spray coating parameters were as follows:

| | |
|---|---|
| Solution Spray Rate | Between 10 gpm (grams per minute) and 17.5 gpm |
| Inlet Temperature | Between 73°C and 76°C |
| Outlet Temperature | Between 44.6°C and 45.7°C |
| Fluidization Air Flow | Between 79.7 cfm (cubic feet per minute) and 81.4 cfm |
| Atomization Air Pressure | 40 psi |

1178 grams of product was harvested.

**Spray 3:** 1178 grams of the enzyme granules from spray 2 were loaded into a Vector FL-1 fluid bed coater and fluidized. 1127 grams of an aqueous solution containing 78 grams (5.4% of granule composition) of poly vinyl alcohol (5/88 from Erkol), 78 grams (5.4% of granule composition) of titanium dioxide (R902 from DuPont), 20 grams (1.35% of granule composition) of talc (Nytal 400) and 20 grams (1.35% of granule composition) of Neodol (23-6.5 from Shell Chemicals) were then sprayed coated onto the enzyme granules. The spray coating parameters were as follows:

| | |
|---|---|
| Solution Spray Rate | 5.2 gpm (grams per minute), increasing to 12.2 gpm over 30 minutes. Kept at 12.2 gpm for remainder of experiment. |
| Inlet Temperature | 70°C, increased to 76°C after 15 minutes |
| Outlet Temperature | Between 51.0°C and 52.5°C |
| Fluidization Air Flow | Between 80.7 cfm (cubic feet per minute) and 84.2 cfm |

(continued)

(continued)

| | |
|---|---|
| Atomization Air Pressure | Between 40 psi and 42 psi |

1356 grams of product was harvested.

**Spray 4:** 1356 grams of the enzyme granules from spray 3 were loaded into a Vector FL-1 fluid bed coater and fluidized. 67.0 grams of an aqueous solution containing 3 grams (0.22% of granule composition) of Neodol (23-6.5 from Shell Chemicals) were then sprayed coated onto the enzyme granules. The spray coating parameters were as follows:

| | |
|---|---|
| Solution Spray Rate | 17.0 gpm over entire spray 4 |
| Inlet Temperature | 76°C |
| Outlet Temperature | Between 44.0°C and 46.5°C |
| Fluidization Air Flow | Between 80.8 cfm (cubic feet per minute) and 81.7cfm |

1359 grams of final product was harvested.

Using the Heubach attrition test, the granules were analyzed for dust. The dust level of the granules was 1.03 mg/pad.

**Example 8**

**Reparation of Enzyme Concentrate with Precipitates and Granular Formulation**

*Preparation of Enzyme Concentrate with Precipitated Enzyme*

Sodium sulfate was added at a concentration 12% (w/v) to the final enzyme concentrate after ultrafiltration in Example 7 to induce precipitation. The concentrate with sodium sulfate was mixed at 10 °C for 18 hours. The resulting slurry was used for was used for granulation.

*Granular Formulation*

A granular composition was prepared from the enzyme concentrate containing precipitated enzyme as follows.

**Spray 1:** 500 grams of sodium sulfate crystal (Saltec), with a particle diameter size range of 150 μm to 350 μm, was loaded into a Vector Fl-1 fluid bed coater and fluidized. To this, 999 grams of solution containing an active protease, 0.7% talc (Nytal 400), 0.9% anti-foam (Foamblast 882 from Emerald Performance Materials), and 0.5% of poly vinyl alcohol (5/88 from Erkol), was spray-coated onto the sodium sulfate crystals. The spray coating parameters were as follows:

| | |
|---|---|
| Solution Spray Rate | 4.5 gpm (grams per minute), increasing to 10.6 gpm over 30 minutes |
| Inlet Temperature | Between 69.0°C and 74.0°C |
| Outlet Temperature | Between 44.5°C and 47.1°C |
| Fluidization Air Flow | Between 79.8 cfm (cubic feet per minute) and 82.2 cfm |
| Atomization Air Pressure | 31 psi (pounds per square inch), increasing to 40 psi over 60 minutes. |

705 grams of product was harvested.

**Spray 2:** 705 grams of the enzyme granules from spray 1 were loaded into a Vector FL-1 fluid bed coater and fluidized. 1847 grams of an aqueous solution containing 369 grams of sodium sulfate (27.7 % of granule composition) were then spray coated onto the enzyme granules.

The spray coating parameters were as follows:

| | |
|---|---|
| Solution Spray Rate | Between 15.2 gpm (grams per minute) and 18.2 gpm |
| Inlet Temperature | Between 70°C and 78°C |
| Outlet Temperature | Between 42.9°C and 45.1°C |
| Fluidization Air Flow | Between 80.2 cfm (cubic feet per minute) and 81.2 cfm |

(continued)

| Atomization Air Pressure | 40 psi |
|---|---|

1061 grams of product was harvested.

**Spray 3:** 1061 grams of the enzyme granules from spray 2 were loaded into a Vector FL-1 fluid bed coater and fluidized. 1000 grams of an aqueous solution containing 72 grams (5.4% of granule composition) of poly vinyl alcohol (5/88 from Erkol), 72 grams (5.4% of granule composition) of titanium dioxide (R902 from DuPont), 18 grams (1.35% of granule composition) of talc (Nytal 400) and 18 grams (1.35% of granule composition) of Neodol (23-6.5 from Shell Chemicals) were then sprayed coated onto the enzyme granules. The spray coating parameters were as follows:

| Solution Spray Rate | 3.8 gpm (grams per minute), increasing to 9.9 gpm over 60 minutes. Kept at 9.9 gpm for remainder of experiment. |
|---|---|
| Inlet Temperature | 72°C, increased to 84°C after 15 minutes |
| Outlet Temperature | Between 51.4°C and 52.9°C |
| Fluidization Air Flow | Between 80.4 cfm (cubic feet per minute) and 82.3 cfm |
| Atomization Air Pressure | Between 40 psi and 42 psi |

1226 grams of product was harvested.

**Spray 4:** 1226 grams of the enzyme granules from spray 3 were loaded into a Vector FL-1 fluid bed coater and fluidized. 62.0 grams of an aqueous solution containing 3 grams (0.22% of granule composition) of Neodol (23-6.5 from Shell Chemicals) were then sprayed coated onto the enzyme granules. The spray coating parameters were as follows:

| Solution Spray Rate | 17.0 gpm over entire spray 4 |
|---|---|
| Inlet Temperature | 69°C |
| Outlet Temperature | Between 41.4°C and 42.3°C |
| Fluidization Air Flow | Between 80.8 cfm (cubic feet per minute) and 80.5cfm |

1228 grams of final product was harvested.

Using the Heubach attrition test, the granules were analyzed for dust. The dust level of the granules was 2.50 mg/pad.

## Example 9

### Preparation of Lysed Bacterial Broth with Enzyme Precipitates and Granular Formulation

#### Preparation of Bacterial Broth Lysate with Precipitated Enzyme

*Bacillus subtilis* that expresses subtilisin protease as described in Example 7 was fermented using well-known techniques in the art. After fermentation, the broth contained mostly soluble subtilisin enzyme. Lysozyme was added to the broth at a concentration of 1% was added to the broth and held at 37°C for 3 hr. The resulting lysate, containing both soluble and insoluble enzyme, contained 57 g/mL activity and 18.23% dry solids.

#### Granular Formulation

A granular composition was prepared from the lysate with precipitate enzyme as follows.

**Spray 1:** 585 grams of sodium sulfate crystal (Saltec), with a particle diameter size range of 150 µm to 350 µm, was loaded into a Vector Fl-1 fluid bed coater and fluidized. To this, 993 grams of solution containing an active protease, 0.7% talc (Nytal 400), 0.9% anti-foam (Foamblast 882 from Emerald Performance Materials), and 0.5% of poly vinyl alcohol (5/88 from Erkol), was spray-coated onto the sodium sulfate crystals. The spray coating parameters were as follows:

| Solution Spray Rate | 4.5 gpm (grams per minute), increasing to 12.5 gpm over 2 hours |
|---|---|
| Inlet Temperature | Between 63°C and 70°C |
| Outlet Temperature | Between 43.0°C and 47.5°C |

(continued)

| | |
|---|---|
| Fluidization Air Flow | Between 79.8 cfm (cubic feet per minute) and 81.1 cfm |
| Atomization Air Pressure | 31 psi (pounds per square inch), increasing to 38 psi over 30 minutes. |

785 grams of product was harvested.

**Spray 2:** 785 grams of the enzyme granules from spray 1 were loaded into a Vector FL-1 fluid bed coater and fluidized. 2001 grams of an aqueous solution containing 400 grams of sodium sulfate (27.7 % of granule composition) were then spray coated onto the enzyme granules. The spray coating parameters were as follows:

| | |
|---|---|
| Solution Spray Rate | Between 15.2 gpm (grams per minute) and 21.2 gpm |
| Inlet Temperature | Between 65°C and 78°C |
| Outlet Temperature | Between 44.1°C and 45.6°C |
| Fluidization Air Flow | Between 78.5 cfm (cubic feet per minute) and 81.6 cfm |
| Atomization Air Pressure | Between 39 psi and 40 psi |

1168 grams of product was harvested.

**Spray 3:** 1168 grams of the enzyme granules from spray 2 were loaded into a Vector FL-1 fluid bed coater and fluidized. 1083 grams of an aqueous solution containing 78 grams (5.4% of granule composition) of poly vinyl alcohol (5/88 from Erkol), 78 grams (5.4% of granule composition) of titanium dioxide (R902 from DuPont), 20 grams (1.35% of granule composition) of talc (Nytal 400) and 20 grams (1.35% of granule composition) of Neodol (23-6.5 from Shell Chemicals) were then sprayed coated onto the enzyme granules. _The spray coating parameters were as follows:

| | |
|---|---|
| Solution Spray Rate | 3.0 gpm (grams per minute), increasing to 8.0 gpm over 60 minutes. Kept at 8.0 gpm for remainder of experiment. |
| Inlet Temperature | 73°C, increased to 78°C after 30 minutes |
| Outlet Temperature | Between 52.0°C and 52.9°C |
| Fluidization Air Flow | Between 80.3 cfm (cubic feet per minute) and 83.7 cfm |
| Atomization Air Pressure | Between 40 psi and 42 psi |

1347 grams of product was harvested.

**Spray 4:** 1347 grams of the enzyme granules from spray 3 were loaded into a Vector FL-1 fluid bed coater and fluidized. 67.0 grams of an aqueous solution containing 3 grams (0.22% of granule composition) of Neodol (23-6.5 from Shell Chemicals) were then sprayed coated onto the enzyme granules. The spray coating parameters were as follows:

| | |
|---|---|
| Solution Spray Rate | 12.6 gpm over entire spray 4 |
| Inlet Temperature | 63°C |
| Outlet Temperature | Between 41.1°C and 44.1°C |
| Fluidization Air Flow | Between 80.9 cfm (cubic feet per minute) and 81.5cm |

1349 grams of final product was harvested.

Using the Heubach attrition test, the granules were analyzed for dust. The dust level of the granules was 1.4 mg/pad.

## Example 10

### Preparation of Fungal Broth Concentrate with Soluble Enzyme and Granular Formulation

#### Preparation of Concentrated Fungal Fermentation Broth with Soluble Enzyme

*Trichoderma reseei* that expresses glucoamylase was fermented using well-known techniques in the art. After fermentation, the broth contained mostly soluble gluco-amylase enzyme. Glucoamylase activity was measured as described in U.S. Patent No. 7,262,041. The cells were removed by microfiltration as described in U.S. Patent Application No. 2007/0246406. The clarified broth was concentrated 10x using a 10K MWCO PES Spiral would membrane. The enzyme concentrate was stored at 10°C until ready for use. Upon storage, precipitates formed. The precipitates were removed by centrifugation (10,000 g, 20min, 10°C). The resulting clear supernatant was used for preparation of a granular composition as described below.

*Granular Formulation*

A granular composition was prepared from the soluble enzyme in fungal fermentation broth as follows.

**Spray 1**: 466 grams of sodium sulfate crystal (Saltec), with a particle diameter size range of 150 μm to 350 μm, was loaded into a Vector FI-1 fluid bed coater and fluidized. To this, 512 grams of solution containing an active amylase, 5% corn starch (Cargill Foods) and 1% of poly vinyl alcohol (5/88 from Erkol), was spray-coated onto the sodium sulfate crystals. The spray coating parameters were as follows:

| | |
|---|---|
| Solution Spray Rate | 8.2 gpm (grams per minute), increasing to 10.1 gpm over 15 minutes. 10.1 gpm was held for the remainder of the experiment. |
| Inlet Temperature | Between 71°C and 77°C |
| Outlet Temperature | Between 46.7°C and 49.1°C |
| Fluidization Air Flow | Between 75.3 cfm (cubic feet per minute) and 80.7 cfm |
| Atomization Air Pressure | 31 psi (pounds per square inch), increasing to 41 psi over 45 minutes. |

615 grams of product was harvested.

**Spray 2:** 615 grams of the enzyme granules from spray 1 were loaded into a Vector FL-1 fluid bed coater and fluidized. 2667 grams of an aqueous solution containing 533 grams of sodium sulfate (40 % of granule composition) were then spray coated onto the enzyme granules. The spray coating parameters were as follows:

| | |
|---|---|
| Solution Spray Rate | Between 15.4 gpm (grams per minute) and 18.5 gpm |
| Inlet Temperature | Between 72°C and 78°C |
| Outlet Temperature | Between 43.8°C and 45.7°C |
| Fluidization Air Flow | Between 80.0 cfm (cubic feet per minute) and 81.8 cfm |
| Atomization Air Pressure | 38 psi |

1113 grams of product was harvested.

**Spray 3:** 1113 grams of the enzyme granules from spray 2 were loaded into a Vector FL-1 fluid bed coater and fluidized. 667 grams of an aqueous solution containing 40 grams (3% of granule composition) of poly vinyl alcohol (5/88 from Erkol) and 80 grams (6% of granule composition) of talc (Nytal 400) were then sprayed coated onto the enzyme granules. The spray coating parameters were as follows:

| | |
|---|---|
| Solution Spray Rate | 4.6 gpm (grams per minute), increasing to 11.0 gpm over 60 minutes. |
| Inlet Temperature | 68°C, increased to 82°C after 10 minutes |
| Outlet Temperature | Between 44.2°C and 52.4°C |
| Fluidization Air Flow | Between 82.3 cfm (cubic feet per minute) and 83.5 cfm |
| Atomization Air Pressure | 40 psi |

1221 grams of final product was harvested.

Using the Heubach attrition test, the granules were analyzed for dust. The dust level of the granules was 1.66 mg/pad.

**Example 11**

**Preparation of Fungal Broth with Enzyme Precipitates and Granular Formulation**

*Trichoderma reseel* that expresses gluco-amylase as described in Example 10 was fermented using well-known techniques in the art. After fermentation, the broth contained mostly soluble gluco-amylase enzyme. Cells were killed without lysis as described in U.S. Patent No. 5,801,034. The precipitate obtained from centrifuging the enzyme concentrate from Example 10 was added to the broth with killed cells. The resulting broth contained both soluble and insoluble enzyme. The enzyme-containing broth exhibited 447 U/mL activity and 19.7% (w/w) dry solids . The amount of precipitate present was determined by measuring the activity of the whole sample, and the supernatant (14,000 rpm, 5 min). The data is summarized in Table 1 below. 90% of the activity was present in the supernatant (as soluble enzyme) and 10% was present in precipitated form.

**TABLE 1**

| | Weight (g) | Activity (GAU/ml) | Total Activity (x10³ GAU) |
|---|---|---|---|
| Broth + Ppt | 200 | 447.2 | 89.440 |
| Supernatant | 181 | 445.9 | 80.708 |

### *Granular Formulation*

A granular composition was prepared from the fungal broth containing precipitate enzyme as follows.

**Spray 1:** 472 grams of sodium sulfate crystal (Saltec), with a particle diameter size range of 150 $\mu$m to 350 $\mu$m, was loaded into a Vector Fl-1 fluid bed coater and fluidized. To this, 494 grams of solution containing an active amylase, 5% corn starch (Cargill Foods) and 1% of poly vinyl alcohol (5/88 from Erkol), was spray-coated onto the sodium sulfate crystals. The spray coating parameters were as follows:

| | |
|---|---|
| Solution Spray Rate | 8.2 gpm (grams per minute), increasing to 10.8 gpm over 10 minutes. 10.8 gpm was held for the remainder of the experiment. |
| Inlet Temperature | Between 68°C and 71°C |
| Outlet Temperature | Between 44.7°C and 46.5°C |
| Fluidization Air Flow | Between 78.5 cfm (cubic feet per minute) and 79.4 cfm |
| Atomization Air Pressure | 33 psi (pounds per square inch), increasing to 40 psi over 20 minutes. |

612 grams of product was harvested.

**Spray 2:** 612 grams of the enzyme granules from spray 1 were loaded into a Vector FL-1 fluid bed coater and fluidized. 2667 grams of an aqueous solution containing 533 grams of sodium sulfate (40 % of granule composition) were then spray coated onto the enzyme granules. The spray coating parameters were as follows:

| | |
|---|---|
| Solution Spray Rate | Between 17.2 gpm (grams per minute) and 23.4 gpm |
| Inlet Temperature | Between 74°C and 82°C |
| Outlet Temperature | Between 44.6°C and 45.5°C |
| Fluidization Air Flow | Between 81.2 cfm (cubic feet per minute) and 81.9 cfm |
| Atomization Air Pressure | 38 psi |

1081 grams of product was harvested.

**Spray 3:** 1081 grams of the enzyme granules from spray 2 were loaded into a Vector FL-1 fluid bed coater and fluidized. 667 grams of an aqueous solution containing 40 grams (3% of granule composition) of poly vinyl alcohol (5/88 from Erkol) and 80 grams (6% of granule composition) of talc (Nytal 400) were then sprayed coated onto the enzyme granules. The spray coating parameters were as follows:

| | |
|---|---|
| Solution Spray Rate | 7.2 gpm (grams per minute), increasing to 10.8 gpm over 60 minutes. |
| Inlet Temperature | 78°C, increased to 81°C over 60 minutes |
| Outlet Temperature | Between 52.7°C and 53.1°C |
| Fluidization Air Flow | Between 83.2 cfm (cubic feet per minute) and 83.6 cfm |
| Atomization Air Pressure | 41 psi |

1190 grams of final product was harvested.

Using the Heubach attrition test, the granules were analyzed for dust. The dust level of the granules was 7.9 mg/pad.

### Example 12

**Enzymatic Stability in Detergent for Granules Prepared from Concentrate containing Cell Components and Insoluble Enzyme**

The stability of enzyme granules prepared as described in Example 4 was assessed in various detergent bases: Calgonite 5-1, WFK with Citrate, WFK with Phosphate, and Somat (Henkel). WFK detergent bases are available from wfk Testgewebe GmbH (www.testgewebe.de). The enzyme granules in detergent (2% w/w) were kept at 22°C in closed containers. Residual enzyme activity was measured after storage at time 0, 5 weeks, 2 months and 4 months. The data is summarized in Table 2 below.

**Table 2**

| Enzyme Granule Stability in Detergent | | | | |
|---|---|---|---|---|
| Detergent Base | Day 0 | 5 weeks | 2 months | 4 months |
| Calgonite 5-1 | 100% | 98% | 93% | 96% |
| WFK with Citrate | 100% | 100% | 94% | 96% |
| WFK with Phosphate | 100% | 100% | 96% | 100% |
| Somat | 100% | 89% | 89% | 98% |

### Example 13

**Application Performance of Granules Prepared from Concentrate containing Cell Components and Insoluble Enzyme**

The washing performance of enzyme granules prepared as described in Example 4 was assessed before and after storage in IEC A* containing bleach detergent base The samples were stored in open cups for 1 week at 37°C/70% relative humidity (RH). Application tests with fresh granules and fresh detergent and with granules stored in detergent for 1 week were performed using various stains: CS-26 (corn starch on cotton), CS-27 (potato starch on cotton), CS-28 (rice starch on cotton), CS-29 (tapioca starch on cotton), and EMPA-161 (starch on cotton).

*Wash test*

The wash test was performed in a Miele Novotronic W822 washing machine: program "Normal"/"Cotton", temperature 40°C, water hardness 8.5 GH. Detergent: IEC A* with bleach: dosage 8 g/L (=136 g/wash), including 0.15 g Purafect 4000 E (available from Genencor Division of Danisco US Inc.) and 0.139 g of Example 4 granules.

*Performance measurements*

Measurements were made with a Minolta CR300 Colorimeter: CIELAB L*ab Scale, D65 Standard Illuminate, without UV filter, white background. Soil removal (SR) was calculated using the following formula:

$$\frac{\Delta E_{\text{after wash- before wash}}}{\Delta E_{\text{unsoiled fabric swatch-before wash}}} \times 100 = \%SR$$

Where:

$$\Delta E = sqrt((\Delta L^*)^2 + (\Delta a)^2 + (\Delta b)^2)$$

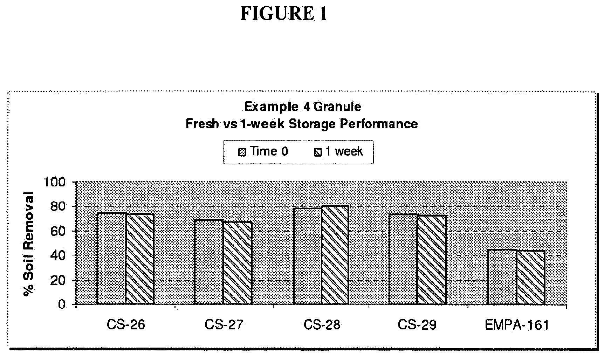

Figure 1 shows that the test performance of Example 4 granules remained unchanged upon storage.

### Example 14

**Enzymatic Stability During Storage for Granules Prepared from Concentrate containing Cell Components and Insoluble Enzyme**

Enzyme granules prepared as described in examples 7-9 were evaluated for storage stability. The conditions used were 37°C in 70% Relative Humidity chamber with samples placed in open containers. Residual activity was measured and calculated based on initial activity. The data is summarized in Table 3 below.

**TABLE 3**

| | | Time (days) | | | | |
|---|---|---|---|---|---|---|
| Sample | | 0 | 3 | 5 | 7 | 14 |
| Example #7 (Granule from Soluble Enzyme) | | 100% | 96% | 97% | 100% | 91% |
| Example #8 (Granule form Precipitated Enzyme) | | 100% | 108% | 109% | 108% | 109% |
| Example #9 (Granule from Precipitated Enzyme Lysate) | | 100% | 96% | 95% | 99% | 96% |

### Example 15

**Comparison of Properties of Recovery Procedures Described in Examples 1-5**

The activity and purity of $\alpha$-amylase recovered as described in the examples 1-5 above is presented in Table 4. Enzyme purity was calculated by dividing the enzyme activity by the total amount of dry solids.

**Table 4**

| Enzyme Lysate Concentrate Properties | | | | | | |
|---|---|---|---|---|---|---|
| Example | Enzyme Purity By Dry Solids | Yield (%) | Relative Waste | Purification Relative to Initial Lysate | Yield relative to Example 1 | Purification Relative to Example 1 |
| 1 | 4.3 | 5% | 0 | 0.6 | 1.0 | 1.0 |
| 2 | 22.1 | 84% | 3% (glucose) | 2.1 | 16.8 | 5.1 |
| 4 | 9.3 | 86% | 0 | 1.4 | 17.1 | 2.2 |
| 5 | 17.2 | 95% | 0 | 1.7 | 19.0 | 4.0 |

### Example 1.6

**Enzymatic Stability During Storage in Detergent Base with Bleach for Granules Prepared from Concentrate containing Cell Components and Insoluble Enzyme**

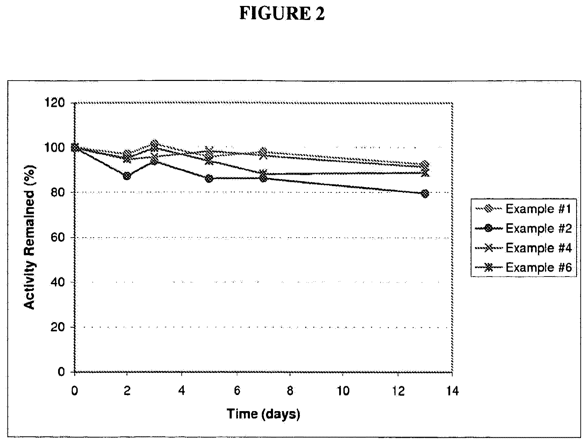

Enzyme granules prepared as described in examples 1, 2, 4 and 6 were evaluated for storage stability in detergent base IEC A* with bleach powder. The IEC A* powder detergent was purchased from WFK and does not contain bleach agents. The following bleach system was added: 3 % TEAD, and 20% perborate.2H2O. The storage conditions used were 37°C in 70% Relative Humidity chamber with samples placed in open polypropylene containers. Residual activity was measured and calculated based on initial activity. The data is shown in Figure 2.

### Example 17

**Preparation of clarified enzyme solution from fermentation broth containing enzyme crystals**

All operations were performed at room temperature.

*Starting Material*

A *Trichoderma reesei* strain engineered to express an aspartic protease was fermented under standard conditions known in the art. For example, see U.S. Patent No. 7,429,476. The pH of the fermentation broth at the time of harvest was 4.62 and the enzyme had formed large crystals with 98% of the total activity in the spindown.

*Filtration 1- Recovery of Protease Crystals*

521 g of harvest broth was blended in a beaker with 63 g of diatomaceous earth filter aid, Celatom FW-6. 302 g of the blend was filtered through a 0.45 μm nylon membrane filter (Nalgene 154-0045). 155 g filtrate and 146 g filter cake were recovered. 98% of the enzyme activity was retained in the filter cake. The filtrate was discarded.

*Crystal Solubilization*

131 g of filter cake from the previous step was transferred into a beaker and blended with 87 g of 50 mM sulfuric acid. After standing for 45 minutes at room temperature, the blend had a pH of 3.2 and a microscope confirmed that all protease crystals had dissolved.

*Filtration 2 - Clarification of Protease Solution*

184 g of the crystal solubilization blend from the previous step was filtered on the same type of filter used in filtration 1. Filtrate was displaced from the sedimented solids by chasing with 50 g of water when the cake surface fell dry. 154 g of filtrate was collected and 80 g of filter cake were discarded. The clear filtrate contained 88% of the starting enzyme activity at a titer that was 16 % higher than that of the starting broth.

## Example 18

**Clarified Enzyme Formulation Preparations**

***Starting Material: Bacterial Fermentation Broth***

*Bacillus licheniformis* that recombinantly expresses α-amylase enzyme was fermented using well-known techniques in the art. After fermentation, the broth was lysed using 0.01 % (w/w) lysozyme for 2 hrs, followed by heat treatment at 60°C for 2hr.

*18.1 Preparation from Broth containing Soluble Enzyme (conventional recovery)*

5g of starting material was centrifuged at 14,000 rpm for 10 min to separate the solid phase from the liquid phase. The supernatant contained 88% of the enzyme activity. The purity by dry solids was 6%. The purity by total protein was 14%.

*18.2 Preparation from Broth containing Insoluble Enzyme*

Crystallization of α-Amylase Enzyme in Lysate

40g of the starting material was used. The pH of the lysate was adjusted to 5 using 20% acetic acid. The pH adjusted was incubated in a shaker: 50°C and 80rpm shaking.

Centrifugation #1 - Recovery of α-Amylase Crystal and cell debris

After 44 hours of incubation, 5 g of lysate was centrifuged at 14,000 rpm for 10 min to separate the solid phase from the liquid phase.

Solubilization of α-Amylase Crystals

The solids recovered were resuspended in tap water to reach the original lysate weight of 5 g. 3 g of propylene glycol was added to the suspension. The pH was adjusted to 6.5 using 5% NaOH and mixed by inversion at room temperature for 18 hr. The mixture was then incubated at 40°C for 2 hrs.

*Centrifugation #2 - Clarification of Amylase Solutions*

The crystal solubilization blend was centrifuged at 14,000 rpm for 10 min to separate the solid phase from the liquid phase. The resulting centrate (liquid supernatant) was further clarified using a $0.45\mu m$ syringe filter. The clear amylase solution contained 85% of the activity found in initiate lysate used for crystallization. The purity by dry solids was 42%. The purity by total protein was 34%.

### 18.3 Preparation from Broth containing Insoluble Enzyme using Flocculation

*Flocculalion*

10g/L of C581 cationic polymer was added to 5 g lysate containing dissolved $\alpha$-amylase crystals from Example 18.2 and then mixed gently by manual inversions 10 times.

*Centrifugation #2 - Clarification of Amylase Solutions*

The flocculated crystal solubilization blend was centrifuged at 14,000 rpm for 10 min to separate the solid phase from the liquid phase. The clear centrate contains 85% of the activity found in initiate lysate used for crystallization. The purity by total protein was 39%.

The purities of $\alpha$-amylase solutions recovered as described in the examples 18.1, 18.2, and 18.3 are presented in Table 5. Enzyme purity was calculated by dividing the enzyme activity by the total amount of dry solids or by dividing by the total protein concentration.

**Table 5: Clarified Enzyme Solution Properties**

| Example | Enzyme Purity by Dry Solids | Purification Relative to Example 18.1 | Enzyme Purity by Total Protein | Purification Relative to Example 18.1 |
|---|---|---|---|---|
| 18.1 | 6% | 1.0 | 14% | 1.0 |
| 18.2 | 42%* | 7.4 | 34% | 2.4 |
| 18.3 | | | 39% | 2.7 |
| *Excluding contribution by popylene glycol | | | | |

**Claims**

1. A method of making an enzyme-containing granular formulation, comprising:

   recovering insoluble enzyme from a microbial broth comprising microbial cells that express the enzyme and/or cell debris from microbial cells that express the enzyme, without removing the microbial cells and/or cell debris, thereby producing a composition comprising recovered insoluble enzyme and microbial cells and/or cell debris, wherein at least some of the enzyme is insoluble in the microbial broth;
   producing an enzyme-containing granule that comprises said composition, wherein the enzyme is enzymatically active in the granule.

2. A method according to claim 1, wherein the enzyme is a hydrolase, a protease, an amylase, a lipase or a cellulase, optionally wherein the enzyme is an $\alpha$-amylase or subtilisin.

3. A method according to claim 1 or claim 2, wherein the enzyme-containing granule is produced by a granulation process selected from top-spray fluid bed processing; bottom spray Wurster coater processing; high shear granulation; extrusion; and pan coating.

4. A method according to any one of claims 1 to 3, comprising coating an enzyme-containing layer comprising said insoluble enzyme and microbial cells and/or cell debris onto a core in a top-spray fluid bed processor, wherein said method optionally comprises coating a salt layer between said core and said enzyme-containing layer, and wherein, optionally, the method further comprises coating a layer comprising a barrier salt over said enzyme-containing layer, optionally further comprising coating an outer coating layer comprising a polymer and/or pigment over said barrier salt layer.

5. A method according to any one of claims 1 to 3, wherein said enzyme containing granule is produced by coating a barrier salt layer and/or one or more coating layers over an enzyme-containing core comprising said insoluble enzyme and microbial cells and/or cell debris, wherein, optionally, said enzyme-containing core is produced by:

   (i) top-spray fluid bed processing, or
   (ii) high shear granulation.

6. A method according to any one of claims 1 to 5, wherein the microbial cells are fungal cells or bacterial cells.

7. The method according to claim 6, wherein the fungal cells are *Trichoderma* cells.

8. The method according to claim 6, wherein the bacterial cells are *Bacillus* cells.


**Patentansprüche**

1. Verfahren zur Herstellung einer enzymhältigen granulären Formulierung, das Folgendes umfasst:

   Gewinnen eines unlöslichen Enzyms aus einer mikrobiellen Kulturlösung, die mikrobielle Zellen, die das Enzym exprimieren, und/oder Zellbruchstücke von mikrobiellen Zellen, die das Enzym exprimieren, umfasst, ohne dass die mikrobiellen Zellen und/oder Zellbruchstücke entfernt werden, wodurch eine Zusammensetzung hergestellt wird, die gewonnenes unlösliches Enzym und mikrobielle Zellen und/oder Zellbruchstücke umfasst, wobei zumindest ein Teil des Enzyms in der mikrobiellen Kulturlösung unlöslich ist;
   Herstellen eines enzymhältigen Granulats, das die Zusammensetzung umfasst, wobei das Enzym im Granulat enzymatisch aktiv ist.

2. Verfahren nach Anspruch 1, wobei das Enzym eine Hydrolase, eine Protease, eine Amylase, eine Lipase oder eine Cellulase ist, wobei das Enzym gegebenenfalls eine $\alpha$-Amylase oder Subtilisin ist.

3. Verfahren nach Anspruch 1 oder Anspruch 2, wobei das enzymhältige Granulat durch einen Granulationsvorgang hergestellt wird, der aus Top-Spray-Wirbelschichtverarbeitung; Bottom-Spray-Wurster-Beschichtungsverarbeitung; Hochschergranulierung; Extrusion; und Trommelbeschichtung ausgewählt ist.

4. Verfahren nach einem der Ansprüche 1 bis 3, welches das Aufbringen einer enzymhältigen Schicht, die ein unlösliches Enzym und mikrobielle Zellen und/oder Zellbruchstücke umfasst, auf einen Kern in einem Top-Spray-Fließbettverarbeitungsgerät umfasst, wobei das Verfahren gegebenenfalls das Aufbringen einer Salzschicht zwischen dem Kern und der enzymhältigen Schicht umfasst
und wobei das Verfahren gegebenenfalls weiters das Aufbringen einer Schicht, die ein Barrieresalz umfasst, auf die enzymhältige Schicht umfasst und gegebenenfalls weiters das Aufbringen einer äußeren Beschichtungsschicht, die ein Polymer und/oder Pigment umfasst, auf die Barrieresalzschicht umfasst.

5. Verfahren nach einem der Ansprüche 1 bis 3, wobei das enzymhältige Granulat durch Aufbringen einer Barrieresalzschicht und/oder einer oder mehrerer Beschichtungsschichten auf den enzymhältigen Kern, der das unlösliche Enzym und mikrobielle Zellen und/oder Zellbruchstücke umfasst, hergestellt wird, wobei der enzymhältige Kern gegebenenfalls durch Folgendes hergestellt wird:

   (i) Top-Spray-Fließbettverarbeitung; oder
   (ii) Hochschergranulierung.

6. Verfahren nach einem der Ansprüche 1 bis 5, wobei die mikrobiellen Zellen Pilzzellen oder Bakterienzellen sind.

7. Verfahren nach Anspruch 6, wobei die Pilzzellen Trichoderma-Zellen sind.

8. Verfahren nach Anspruch 6, wobei die Bakterienzellen Bacillus-Zellen sind.

**Revendications**

1. Méthode pour la fabrication d'une formulation granulaire contenant une enzyme, comprenant :

la récupération d'une enzyme insoluble a partir d'un bouillon microbien comprenant des cellules microbiennes qui expriment l'enzyme et/ou des débris cellulaires provenant des cellules microbiennes qui expriment l'enzyme, sans le retrait des cellules microbiennes et/ou des débris cellulaires, produisant ainsi une composition comprenant l'enzyme insoluble récupérée et des cellules microbiennes et/ou des débris cellulaires, dans laquelle au moins une partie de l'enzyme est insoluble dans le bouillon microbien ;
la production d'un granulé contenant une enzyme qui comprend ladite composition, dans laquelle l'enzyme est enzymatiquement active dans le granulé.

2. Méthode selon la revendication 1, dans laquelle l'enzyme est une hydrolase, une protéase, une amylase, une lipase ou une cellulase, optionnellement dans laquelle l'enzyme est une α-amylase ou une subtilisine.

3. Méthode selon la revendication 1 ou la revendication 2, dans laquelle le granulé contenant une enzyme est produit par un procédé de granulation choisi parmi un traitement par lit fluidisé à pulvérisation supérieure ; un traitement par dispositif de revêtement Wurster à pulvérisation inférieure ; une granulation a cisaillement élevé ; une extrusion ; et un revêtement au tambour.

4. Méthode selon l'une quelconque des revendications 1 à 3, comprenant le revêtement d'une couche contenant une enzyme comprenant ladite enzyme insoluble et des cellules microbiennes et/ou des débris cellulaires sur un noyau dans un appareil de traitement par lit fluidisé à pulvérisation supérieure, dans laquelle ladite méthode comprend optionnellement le revêtement d'une couche de sel entre ledit noyau et ladite couche contenant une enzyme, et dans laquelle, optionnellement, la méthode comprend en outre le revêtement d'une couche comprenant un sel barrière au-dessus de ladite couche contenant une enzyme, optionnellement comprenant en outre le revêtement d'une couche de revêtement externe comprenant un polymère et/ou un pigment au-dessus de ladite couche de sel barrière.

5. Méthode selon l'une quelconque des revendications 1 à 3, dans laquelle ledit granulé contenant une enzyme est produit par le revêtement d'une couche de sel barrière et/ou d'une ou de plusieurs couches de revêtement au-dessus d'un noyau contenant une enzyme comprenant ladite enzyme insoluble et des cellules microbiennes et/ou des débris cellulaires, dans laquelle, optionnellement, ledit noyau contenant une enzyme est produit par :

(i) un traitement par lit fluidisé à pulvérisation supérieure, ou
(ii) une granulation a cisaillement élevé.

6. Méthode selon l'une quelconque des revendications 1 a 5, dans laquelle les cellules microbiennes sont des cellules fongiques ou des cellules bactériennes.

7. Méthode selon la revendication 6, dans laquelle les cellules fongiques sont des cellules de *Trichoderma.*

8. Méthode selon la revendication 8, où les cellules bactériennes sont des cellules de *Bacillus.*

# FIGURE 1

Example 4 Granule
Fresh vs 1-week Storage Performance

☒ Time 0   ☒ 1 week

# FIGURE 2

## REFERENCES CITED IN THE DESCRIPTION

### Patent documents cited in the description

- WO 0187468 A
- WO 9109941 A
- EP 1417301 A
- US 4760025 A
- EP 130756 A
- WO 91106637 A
- WO 08112459 A
- WO 05056782 A
- US 5801034 A
- US 5324649 A
- US 5879920 A
- US 7108821 B
- US 4689287 A
- WO 0024877 A
- WO 9106637 A
- US 9200384 W
- EP 1037968 B1
- US 5576282 A
- US 6306812 B
- US 6326348 B
- US 4430243 A
- US 5597936 A
- US 5595967 A
- WO 00332601 A
- US 6225464 B
- US 041917
- WO 0811245 A
- US 7262041 B
- US 20070246406 A
- US 7429476 B

### Non-patent literature cited in the description

- Enzyme in Industry: Production and Applications. Wiley-VCH, 2007, 49
- **R.G. HARRISON ; P. TODD ; S.R. RUDGEI ; D.P. PETRIDES.** Bioseparations: Science and Engineering. Oxford University Press, 2003, 32
- Isolation and Purification of Proteins. Marcell Dekker, Inc, 2003, 1-27
- **BARAN.** *Colloids Surf,* 1988, vol. 31, 259-264
- **BAUTISTA et al.** *Biotechnol. Lett,* 1986, vol. 8, 315-318
- **CHEN ; BERG.** *Chem. Eng. Sci,* 1993, vol. 48, 1775-1784
- **CUMMING et al.** *Biotechnol. Tech,* 1996, vol. 4, 55-60
- **HUSTEDT ; THEELEN.** DeChema Biotechnology Conferences. VCH Veragsgesselschaft, 1989, vol. 3, 1071-1075
- **RAMSDEN et al.** *Biotechnol. Tech,* 1998, vol. 12, 599-603
- **SHAN et al.** *J. Biotechnol,* 1996, vol. 49, 173-178
- **SAMBROOK et al.** Molecular Cloning: A Laboratory Manual. 1989
- Oligonucleotide Synthesis. 1984
- Current Protocols in Molecular Biology. 1994
- PCR: The Polymerise Chain Redaction. 1994
- **KRIEGLER.** Gene Transfer and Expression: A Laboratory Manual. 1990
- **SINGLETON et al.** Dictionary of Microbiology and Molecular Biology. John Wiley and Sons, 1994
- **HALE ; MARKHAM.** The Harper Collins Dictionary of Biology. Harper Perennial, 1991
- **ALEXOPOULOS, C. J.** INTRODUCTORY MYCOLOGY. Wiley, 1962
- **INNIS et al.** *Sci.,* 1985, vol. 228, 21-26
- Isolation and Purification of Proteins. Marcel Dekker, Inc, 2003, 225-275
- **R.G. HARRISON ; P. TODD ; S.R. RUDGE ; D.P. PETRIDES.** *Bioseparations: Science and Engineering,* 2003, 243-271
- **R.K. SCOPES.** Protein Purification: Principles and Practice. Springer, 1994, 71-101
- Differential Precipitation of Proteins. Protein Purification Process Engineering. Marcel Dekker Inc, 1994, 115-208
- Enzymes In Detergency. Marcel Dekker, Inc, 1997, 310-312
- **VARDAR-SUKAN.** *Recent Adv. Biotechnol,* 1991, 113-146
- **GHILDYAL.** *Adv. Appl. Microbiol,* 1988, vol. 33, 173-222